(19) Europäisches Patentamt
European Patent Office
Office européen des brevets

(11) EP 2 247 752 B1

(12) EUROPEAN PATENT SPECIFICATION

(45) Date of publication and mention of the grant of the patent:
**05.11.2014 Bulletin 2014/45**

(21) Application number: **09708938.7**

(22) Date of filing: **04.02.2009**

(51) Int Cl.:
***C12Q 1/68*** *(2006.01)*

(86) International application number:
**PCT/US2009/033091**

(87) International publication number:
**WO 2009/100141 (13.08.2009 Gazette 2009/33)**

(54) **PREDICTION AND DIAGNOSIS OF CANINE DEGENERATIVE MYELOPATHY**

VORHERSAGE UND DIAGNOSE VON DEGENERATIVER MYELOPATHIE BEI HUNDEN

PRÉDICTION ET DIAGNOSTIC DE LA MYÉLOPATHIE DÉGÉNÉRATIVE CANINE

(84) Designated Contracting States:
**AT BE BG CH CY CZ DE DK EE ES FI FR GB GR HR HU IE IS IT LI LT LU LV MC MK MT NL NO PL PT RO SE SI SK TR**

(30) Priority: **04.02.2008 US 25949**

(43) Date of publication of application:
**10.11.2010 Bulletin 2010/45**

(73) Proprietors:
- **The Curators of The University of Missouri Columbia, MO 65211 (US)**
- **Massachusetts Institute of Technology Cambridge, MA 02139 (US)**
- **Massachusetts General Hospital Boston, MA 02114 (US)**

(72) Inventors:
- **COATES, Joan, R, Columbia MO 65211 (US)**
- **LINBLAD-TOH, Kerstin Malden MA 02148 (US)**
- **WADE, Claire Como NSW 2226 (AU)**
- **JOHNSON, Gary, S. Columbia MO 65211 (US)**

(74) Representative: **Potter Clarkson LLP The Belgrave Centre Talbot Street Nottingham, NG1 5GG (GB)**

(56) References cited:
- **AWANO TOMOYUKI ET AL: "Genome-wide association analysis reveals a SOD1 mutation in canine degenerative myelopathy that resembles amyotrophic lateral sclerosis." PROCEEDINGS OF THE NATIONAL ACADEMY OF SCIENCES OF THE UNITED STATES OF AMERICA 24 FEB 2009, vol. 106, no. 8, 24 February 2009 (2009-02-24), pages 2794-2799, XP002527026 ISSN: 1091-6490**
- **JOHNSON ET AL: "A SOD1 missense mutation in dogs with degenerative myelopathy: a spontaneous amyotrophic lateral sclerosis model"[Online] November 2008 (2008-11), page 1, XP002527027 Retrieved from the Internet: URL: http://www.ashg.org/2008meeting/abstra cts/ fulltext/f20625.htm> [retrieved on 2009-05-06]**
- **O'BRIEN ET AL: "SOD1 immunoreactivity in a spontaneous canine model of amyotrophic lateral sclerosis (ALS)"[Online] 2008, page 1, XP002527028 Retrieved from the Internet: URL: http://som.missouri.edu/ophthalmology/ Faculty/katz-m/DM%20SOD1%20immuno%20SFN%20 11-08%20MK%20B.pdf> [retrieved on 2009-05-06]**
- **SANDELIN ERIK ET AL: "Amyotrophic lateral sclerosis-associated copper/zinc superoxide dismutase mutations preferentially reduce the repulsive charge of the proteins." THE JOURNAL OF BIOLOGICAL CHEMISTRY 20 JUL 2007, vol. 282, no. 29, 20 July 2007 (2007-07-20), pages 21230-21236, XP002527029 ISSN: 0021-9258 cited in the application**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 247 752 B1

- GREEN S L ET AL: "Structure, chromosomal location, and analysis of the canine Cu/Zn superoxide dismutase (SOD1) gene" JOURNAL OF HEREDITY 2002 US, vol. 93, no. 2, 2002, pages 119-124, XP002527030 ISSN: 0022-1503
- VENTURA DOG BLOG: "Interesting Information on Degenerative Myelopathy"[Online] 2007, page 1, XP002527031 Retrieved from the Internet: URL: http://venturahomesfordogs.blogspot.co m/ 2007/10/interesting-information-on-degen erative.html> [retrieved on 2009-05-06]

SEQUENCE LISTING

<110> COATES, JOAN R. LINBLAD-TOH, KERSTIN WADE, CLAIRE JOHNSON, GARY S.

<120> PREDICTION AND DIAGNOSIS OF CANINE DEGENERATIVE MYELOPATHY

<130> UVMO:047WO

<140> UNKNOWN
<141> 2009-02-04

<150> 61/025,949
<151> 2008-02-04

<160> 30

<170> PatentIn version 3.5

<210> 1

<211> 94
<212> DNA
<213> Artificial

<220>
<223> Synthetic primer

<400> 1

```
ggaagtgggc ctgttgtggt atcaggaacc attacagggc tgactaaagg cgagcatgga       60

ttccacgtcc atcagtttgg agataataca caag                                   94
```

<210> 2
<211> 94
<212> DNA
<213> Artificial

<220>
<223> Synthetic primer

<400> 2

```
ggaagtgggc ctgttgtggt atcaggaacc attacagggc tgactaaagg cgagcatgga       60

ttccacgtcc atcagtttgg agataataca caag                                   94
```

<210> 3
<211> 21
<212> DNA
<213> Artificial

<220>
<223> Synthetic primer

<400> 3
agtgggcctg ttgtggtatc a 21

<210> 4
<211> 23
<212> DNA
<213> Artificial

<220>
<223> Synthetic primer

<400> 4
ctccaaactg atggacgtgg aat 23

<210> 5
<211> 16
<212> DNA
<213> Artificial

<220>
<223> Synthetic primer

<400> 5
aatccatgct cgcctt 16

<210> 6
<211> 462
<212> DNA
<213> Canis familiaris

<400> 6

```
atggagatga aggccgtgtg cgtgttgaag ggccagggcc cggtggaggg caccatccac      60
ttcgtgcaga agggaagtgg gcctgttgtg gtatcaggaa ccattacagg gctgactgaa     120
ggcgagcatg gattccacgt ccatcagttt ggagataata cacaaggctg tactagtgca     180
ggtcctcact ttaatcctct gtccaaaaaa catggtgggc caaaagatca agagaggcat     240
gttggagacc tgggcaatgt gactgctggc aaggatggcg tggccattgt gtccatagaa     300
gattctctga ttgcactctc aggagactat tccatcattg gccgcaccat ggtggtccac     360
gagaaacgag atgacttggg caaaggtgac aatgaagaaa gtacacagac aggaaacgcc     420
gggagtcgtt tggcttgtgg tgtcattggg atcgccaagt aa                        462
```

<210> 7
<211> 462
<212> DNA
<213> Canis familiaris

<400> 7

```
atggagatga aggccgtgtg cgtgttgaag ggccagggcc cggtggaggg caccatccac      60
ttcgtgcaga agggaagtgg gcctgttgtg gtatcaggaa ccattacagg gctgactgaa     120
ggcgagcatg gattccacgt ccatcagttt ggagataata cacaaggctg tactagtgca     180
ggtcctcact ttaatcctct gtccaaaaaa catggtgggc caaaagatca agagaggcat     240
gttggagacc tgggcaatgt gactgctggc aaggatggcg tggccattgt gtccatagaa     300
gattctctga ttgcactctc aggagactat tccatcattg gccgcaccat ggtggtccac     360
gagaaacgag atgacttggg caaaggtgac aatgaagaaa gtacacagac aggaaacgcc     420
gggagtcgtt tggcttgtgg tgtcattggg atcgccaagt aa                        462
```

<210> 8
<211> 153
<212> PRT
<213> Canis familiaris

<400> 8

```
Met Glu Met Lys Ala Val Cys Val Leu Lys Gly Gln Gly Pro Val Glu
1               5                   10                  15

Gly Thr Ile His Phe Val Gln Lys Gly Ser Gly Pro Val Val Val Ser
            20                  25                  30

Gly Thr Ile Thr Gly Leu Thr Glu Gly Glu His Gly Phe His Val His
        35                  40                  45

Gln Phe Gly Asp Asn Thr Gln Gly Cys Thr Ser Ala Gly Pro His Phe
    50                  55                  60

Asn Pro Leu Ser Lys Lys His Gly Gly Pro Lys Asp Gln Glu Arg His
65                  70                  75                  80

Val Gly Asp Leu Gly Asn Val Thr Ala Gly Lys Asp Gly Val Ala Ile
                85                  90                  95

Val Ser Ile Glu Asp Ser Leu Ile Ala Leu Ser Gly Asp Tyr Ser Ile
            100                 105                 110

Ile Gly Arg Thr Met Val Val His Glu Lys Arg Asp Asp Leu Gly Lys
        115                 120                 125

Gly Asp Asn Glu Glu Ser Thr Gln Thr Gly Asn Ala Gly Ser Arg Leu
    130                 135                 140

Ala Cys Gly Val Ile Gly Ile Ala Lys
145                 150
```

<210> 9
<211> 153
<212> PRT
<213> Canis familiaris

<400> 9

```
Met Glu Met Lys Ala Val Cys Val Leu Lys Gly Gln Gly Pro Val Glu
1               5                   10                  15

Gly Thr Ile His Phe Val Gln Lys Gly Ser Gly Pro Val Val Val Ser
            20                  25                  30

Gly Thr Ile Thr Gly Leu Thr Lys Gly Glu His Gly Phe His Val His
        35                  40                  45

Gln Phe Gly Asp Asn Thr Gln Gly Cys Thr Ser Ala Gly Pro His Phe
    50                  55                  60

Asn Pro Leu Ser Lys Lys His Gly Gly Pro Lys Asp Gln Glu Arg His
65                  70                  75                  80

Val Gly Asp Leu Gly Asn Val Thr Ala Gly Lys Asp Gly Val Ala Ile
                85                  90                  95

Val Ser Ile Glu Asp Ser Leu Ile Ala Leu Ser Gly Asp Tyr Ser Ile
            100                 105                 110

Ile Gly Arg Thr Met Val Val His Glu Lys Arg Asp Asp Leu Gly Lys
        115                 120                 125

Gly Asp Asn Glu Glu Ser Thr Gln Thr Gly Asn Ala Gly Ser Arg Leu
    130                 135                 140

Ala Cys Gly Val Ile Gly Ile Ala Lys
145                 150
```

<210> 10
<211> 41
<212> PRT
<213> Canis familiaris

<400> 10

```
Phe Val Gln Lys Gly Ser Gly Pro Val Val Val Ser Gly Thr Ile Thr
1               5                   10                  15

Gly Leu Thr Glu Gly Glu His Gly Phe His Val His Gln Phe Gly Asp
            20                  25                  30

Asn Thr Gln Gly Cys Thr Ser Ala Gly
        35                  40
```

<210> 11
<211> 42

<212> PRT
<213> Rattus norvegicus

<400> 11

```
Phe Glu Gln Lys Ala Ser Gly Glu Pro Val Val Val Ser Gly Gln Ile
1               5                   10                  15

Thr Gly Leu Thr Glu Gly Glu His Gly Phe His Val His Gln Tyr Gly
            20                  25                  30

Asp Asn Thr Gln Gly Cys Thr Thr Ala Gly
        35                  40
```

<210> 12
<211> 42
<212> PRT
<213> Cavia porcellus

<400> 12

```
Phe Glu Gln Lys Ala Ser Gly Glu Pro Val Val Val Ser Gly Gln Ile
1               5                   10                  15

Thr Gly Leu Thr Glu Gly Glu His Gly Phe His Val His Gln Tyr Gly
            20                  25                  30

Asp Asn Thr Gln Gly Cys Thr Thr Ala Gly
        35                  40
```

<210> 13
<211> 42
<212> PRT
<213> Mus musculus

<400> 13

```
Phe Glu Gln Lys Ala Ser Gly Glu Pro Val Val Leu Ser Gly Gln Ile
1               5                   10                  15

Thr Gly Leu Thr Glu Gly Gln His Gly Phe His Val His Gln Tyr Gly
            20                  25                  30

Asp Asn Thr Gln Gly Cys Thr Ser Ala Gly
        35                  40
```

<210> 14
<211> 41
<212> PRT
<213> Oryctolagus cuniculus

<400> 14

```
Phe Glu Gln Lys Gly Thr Gly Pro Val Val Val Lys Gly Arg Ile Thr
1               5                   10                  15

Gly Leu Thr Glu Gly Leu His Glu Phe His Val His Gln Phe Gly Asp
            20                  25                  30

Asn Arg Gln Gly Cys Thr Ser Ala Gly
        35                  40
```

<210> 15
<211> 42
<212> PRT
<213> Macaca mulatta

<400> 15

```
Phe Glu Gln Lys Glu Ser Asn Gly Pro Val Lys Val Trp Gly Ser Ile
1               5                   10                  15

Thr Gly Leu Thr Glu Gly Leu His Gly Phe His Val His Gln Phe Gly
            20                  25                  30

Asp Asn Thr Gln Gly Cys Thr Ser Ala Gly
        35                  40
```

<210> 16
<211> 42
<212> PRT
<213> Gorilla gorilla

<400> 16

```
Phe Glu Gln Lys Glu Ser Asn Gly Pro Val Lys Val Tyr Gly Arg Ile
1               5                   10                  15

Thr Gly Leu Thr Glu Gly Leu His Gly Phe His Val His Gln Phe Gly
            20                  25                  30

Asp Asn Thr Gln Gly Cys Thr Ser Ala Gly
        35                  40
```

<210> 17
<211> 42
<212> PRT
<213> Cervus elaphus

<400> 17

```
Ile Arg Phe Glu Ala Lys Gly Asn Thr Val Val Val Thr Gly Ser Ile
```

```
1               5                   10                  15

Thr Gly Leu Thr Glu Gly Asp His Gly Phe His Val His Gln Phe Gly
            20                  25                  30

Asp Asn Thr Gln Gly Cys Thr Ser Ala Gly
        35                  40
```

<210> 18
<211> 42
<212> PRT
<213> Bos taurus

<400> 18

```
Ile His Phe Glu Ala Lys Gly Asn Thr Val Val Val Thr Gly Ser Ile
1               5                   10                  15

Thr Gly Leu Thr Glu Gly Asp His Gly Phe His Val His Gln Phe Gly
            20                  25                  30

Asp Asn Thr Gln Gly Cys Thr Ser Ala Gly
        35                  40
```

<210> 19
<211> 42
<212> PRT
<213> Ovis aries

<400> 19

```
Ile Arg Phe Glu Ala Lys Gly Asp Lys Val Val Val Thr Gly Ser Ile
1               5                   10                  15

Thr Gly Leu Thr Glu Gly Asp His Gly Phe His Val His Gln Phe Gly
            20                  25                  30

Asp Asn Thr Gln Gly Cys Thr Ser Ala Gly
        35                  40
```

<210> 20
<211> 42
<212> PRT
<213> Macaca fascicularis

<400> 20

```
Phe Glu Gln Lys Glu Ser Asn Gly Pro Val Lys Val Trp Gly Ser Ile
1               5                   10                  15

Thr Gly Leu Thr Glu Gly Leu His Gly Tyr His Val His Gln Phe Gly
```

20 25 30

Asp Asn Thr Gln Gly Cys Thr Ser Ala Gly
35 40

<210> 21
<211> 42
<212> PRT
<213> Bos grunniens

<400> 21

Ile His Phe Glu Ala Lys Gly Asp Thr Val Val Val Thr Gly Ser Ile
1 5 10 15

Thr Gly Leu Thr Glu Gly Asp His Gly Phe His Val His Gln Phe Gly
20 25 30

Asp Asn Thr Gln Gly Cys Thr Ser Ala Gly
35 40

<210> 22
<211> 42
<212> PRT
<213> Capra hircus

<400> 22

Ile His Phe Glu Ala Lys Gly Asp Lys Val Val Val Thr Gly Ser Ile
1 5 10 15

Thr Gly Leu Thr Glu Gly Asp His Gly Phe His Val His Gln Phe Gly
20 25 30

Asp Asn Thr Gln Gly Cys Thr Ser Ala Gly
35 40

<210> 23
<211> 42
<212> PRT
<213> Sus scrofa

<400> 23

Tyr Phe Glu Leu Lys Gly Glu Lys Thr Val Leu Val Thr Gly Thr Ile
1 5 10 15

Lys Gly Leu Ala Glu Gly Asp His Gly Phe His Val His Gln Phe Gly
20 25 30

Asp Asn Thr Gln Gly Cys Thr Ser Ala Gly

35 40

<210> 24
<211> 42
<212> PRT
<213> Callithrix jacchus

<400> 24

```
Phe Glu Gln Lys Glu Ser Asn Gly Pro Val Lys Val Trp Gly Ser Ile
1               5                   10                  15

Thr Gly Leu Ala Glu Gly Leu His Gly Phe His Val His Gln Phe Gly
            20                  25                  30

Asp Asn Thr Gln Gly Cys Thr Ser Ala Gly
        35                  40
```

<210> 25
<211> 42
<212> PRT
<213> Cebus apella

<400> 25

```
Phe Glu Gln Lys Glu Ser Asn Gly Pro Val Lys Val Trp Gly Ser Ile
1               5                   10                  15

Thr Gly Leu Ala Glu Gly Leu His Gly Phe His Val His Gln Phe Gly
            20                  25                  30

Asp Asn Thr Gln Gly Cys Thr Ser Ala Gly
        35                  40
```

<210> 26
<211> 42
<212> PRT
<213> Monodelphis domestica

<400> 26

```
Phe Glu Gln Lys Gln Val Gly Glu Pro Val Glu Leu Ser Gly Ser Ile
1               5                   10                  15

Lys Gly Leu Ala Glu Gly Asp His Gly Phe His Val His Glu Phe Gly
            20                  25                  30

Asp Asn Thr Gln Gly Cys Thr Ser Ala Gly
        35                  40
```

<210> 27

<211> 42
<212> PRT
<213> Homo sapiens

<400> 27

```
Phe Glu Gln Lys Glu Ser Asn Gly Pro Val Lys Val Trp Gly Ser Ile
1               5                   10                  15

Lys Gly Leu Thr Glu Gly Leu His Gly Phe His Val His Glu Phe Gly
            20                  25                  30

Asp Asn Thr Ala Gly Cys Thr Ser Ala Gly
        35                  40
```

<210> 28
<211> 42
<212> PRT
<213> Pongo pygmaeus

<400> 28

```
Phe Glu Gln Lys Glu Arg Asn Gly Pro Val Lys Val Trp Gly Ser Ile
1               5                   10                  15

Glu Gly Leu Thr Glu Gly Leu His Gly Phe His Val His Glu Phe Gly
            20                  25                  30

Asp Asn Thr Val Gly Cys Thr Ser Ala Gly
        35                  40
```

<210> 29
<211> 42
<212> PRT
<213> Equus caballus

<400> 29

```
Phe Glu Gln Gln Gln Glu Gly Gly Pro Val Val Leu Lys Gly Phe Ile
1               5                   10                  15

Glu Gly Leu Thr Lys Gly Asp His Gly Phe His Val His Glu Phe Gly
            20                  25                  30

Asp Asn Thr Gln Gly Cys Thr Thr Ala Gly
        35                  40
```

<210> 30
<211> 41
<212> PRT
<213> Canis familiaris

<400> 30

```
Phe Val Gln Lys Gly Ser Gly Pro Val Val Val Ser Gly Thr Ile Thr
1               5                   10                  15

Gly Leu Thr Lys Gly Glu His Gly Phe His Val His Gln Phe Gly Asp
            20                  25                  30

Asn Thr Gln Gly Cys Thr Ser Ala Gly
        35                  40
```

## Description

### 1. Field of the Invention

The present invention relates generally to the fields of molecular genetics, veterinary medicine, veterinary neurology, human neurology and human medicine. More particularly, it concerns the veterinary disease canine degenerative myelopathy and the human disease amyotrophic lateral sclerosis. Specifically, the invention relates to the use of genetic markers to identify the presence of a major genetic risk factor for canine degenerative myelopathy which is a fatal veterinary disease and a model for amyotrophic lateral sclerosis (ALS).

### 2. Description of Related Art

Canine degenerative myelopathy (CDM; also known as chronic degenerative radiculomyelopathy) is a neurological disease common in German Shepherds, Welsh Corgis, and several other breeds. The disease is chronic and progressive, and can result in lameness in the animal and eventually may lead to extensive paralysis of the back legs. The animal could be crippled within a few months, or may survive up to 3 years. Under any scenario, the disease is debilating for the animal, severely hampers the quality of life, culminates in euthanasia, and is devastating for pet owners and breeders alike.

CDM is a chronic condition that cannot be cured, though it may be possible to maintain the dog's quality of life for a short time through a proper program of exercise and nutrition. Exercise has been recommended to maintain the dog's ability to walk, and physiotherapy may prolong the length of time that the dog remains mobile and increase survival time, but the ultimate outcome is not favorable. There are still no proven effective therapies to halt or slow progression of CDM. Although not approved by the U.S. Food and Drug Administration (FDA), aminocaproic acid (EACA) and n-acetylcysteine (NAC) may slow the progression, but this treatment is still experimental and controversial.

According to Green et al, 2002, The American Genetic Association 93:119-124, mutations in the SOD1 human gene have been found in the amyotrophic lateral sclerosis (ALS).

Considering the debilitating nature of the disease and the lack of proven therapies, the identification of genetic markers to permit the identification of animals that are predisposed to developing CDM would be very beneficial to breeders and pet owners alike.

### SUMMARY OF THE INVENTION

Thus, in accordance with the present invention, there is provided a method of identifying a dog as having or at risk of developing degenerative myelopathy , or producing offspring having or at risk of developing degenerative myelopathy, comprising (a) providing a nucleic acid-containing sample from said dog; (b) assessing the structure of an SOD1 gene or transcript corresponding to amino acid residue 40 of the corresponding SOD1 polypeptide in said nucleic acid-containing sample from said dog; and (c) identifying said dog as (i) having or at risk of developing degenerative myelopathy when a polymorphism in the SOD1 coding region corresponds to a homozygous E $\rightarrow$ K substitution at amino acid residue 40 of the corresponding SOD1 polypeptide, as compared to a reference wild-type SOD1 gene or transcript is observed, or (ii) producing offspring having or at risk of developing degenerative myelopathy when a polymorphism in the SOD1 coding region corresponds to a heterozygous E $\rightarrow$ K substitution at amino acid residue 40 of the corresponding SOD1 polypeptide, as compared to a reference wild-type SOD1 gene or transcript is observed. The dog may be a boxer, a Welsh corgi, a Chesapeake Bay retriever, a Rhodesian Ridgeback, a German shepherd, a Kerry blue terrier, a Irish setter, a old English sheepdog, a collie, a Standard poodle, a wire Fox terrier, another breed, or a cross-breed thereof. Step (b) may comprise pyrosequencing, competitive probe hybridization, chain-terminating sequencing, restriction digestion, allele-specific polymerase reaction, single-stranded conformational polymorphism analysis, genetic bit analysis, TaqMan® allelic discrimination assay, temperature gradient gel electrophoresis, ligase chain reaction, melting curve profiles, or microarray hybridization, microarray analysis in combination other nucleic acid marker assays, or may comprise assessing the structure of a genetic locus that is in linkage disequilibrium with said genetic marker.

The reference wild-type SOD1 exon 2 sequence may comprise the following sequence:

5'-ggaagtgggcctgttgtggtatcaggaaccattacagggctgactaaaggcgagcatggattccacgtccatcagtttggagata atacacaag-3' (SEQ ID NO:1)

The present application also describes a method of assessing a treatment for canine degenerative myelopathy or amyotrophic lateral sclerosis comprising assessing a candidate therapy for efficacy against said degenerative myelopathy or amyotrophic lateral sclerosis a dog having a mutation in an SOD1 gene as compared to a reference wild-type SOD1 gene or transcript, wherein the dog has been administered the candidate therapy. The dog may be a boxer, a Welsh corgi , a Chesapeake Bay retriever, a Rhodesian Ridgeback, a German shepherd, a Kerry blue terrier, a Irish setter, a old English sheepdog, a Collie, a standard poodle, a wire Fox terrier, or another breed, or a cross-breed thereof. The mutation may be a polymorphism in the SOD1 coding region corresponding to an E→K substitution at residue 40 of the SOD1 polypeptide. Assessing may comprise neurologic examination, electrodiagnostic testing, CT/myelography, MRI, or functional imaging, or assessing may comprise spinal chord immunohistopathology.

It is contemplated that any method or composition described herein can be implemented with respect to any other method or composition described herein.

The use of the word "a" or "an" when used in conjunction with the term "comprising" in the claims and/or the specification may mean "one," but it is also consistent with the meaning of "one or more," "at least one," and "one or more than one."

These, and other, embodiments of the invention will be better appreciated and understood when considered in conjunction with the following description and the accompanying drawings. It should be understood, however, that the following description, while indicating various embodiments of the invention and numerous specific details thereof, is given by way of illustration and not of limitation.

## BRIEF DESCRIPTION OF THE DRAWINGS

The following drawings form part of the present specification and are included to further demonstrate certain aspects of the present invention. The invention may be better understood by reference to one or more of these drawings in combination with the detailed description of specific embodiments presented herein.

**FIGS. 1A-C -** Mapping of a major DM locus. (FIG. 1A) GWA of 49,663 SNPs by using 38 cases (phenotypic stringencies 1 to 4) and 17 controls from Pembroke Welsh corgis identified a major locus on CFA31 ($p_{genome}$ = 0.18) and weaker signals on other chromosomes by using 10,000 permutations in PLINK (38). (FIG. 1B) The CFA31 region of association spans ~1.5 Mb and includes *SOD1. P* values from fine-mapping with 90 SNPs in 63 cases (phenotypic stringency levels 1-3) and 144 controls from 5 breeds (Boxer, 8/15 [Case/Control]; Chesapeake Bay retriever, 9/48; German Shepherd dog, 4/54; Pembroke Welsh corgi, 35/17; and Rhodesian ridgeback, 7/8) are shown as well as the association for the missense mutation, which was separately assayed. (FIG. 1C) Fine-mapping data shows that a 195-kb haplotype surrounding the *SOD1* mutation is associated in all 5 breeds and that this haplotype is older than the *SOD1* mutation.

**FIGS. 2A-B -** Spinal cord histopathology. (FIG. 2A) Luxol fast blue-periodic acid Schiff staining of a thoracic spinal cord cross-section from a DM-affected 13-year-old Pembroke Welsh corgi. The white matter degeneration is depicted by regions of pallor where there has been loss of nerve fibers. (FIG. 2B) A similarly stained spinal cord cross section from an unaffected 13-year-old Labrador retriever. Note there is no evidence of nerve fiber loss. The bar in the lower right of the photomicrograph indicates the magnification.

**FIGS. 3A-I -** Immunohistochemical staining with anti-SOD1 antibody. Representative sections from the spinal cords from 3 G/G homozygous asymptomatic control dogs (FIGS. 3A-C), 3 A/G heterozygous asymptomatic control dogs (FIGS. 3D-F), and 3 A/A homozygous dogs with a confirmed diagnosis of DM (FIGS. 3G-I). The samples were from a 13-year-old Rhodesian ridgeback (FIG. 3A), an 8-year-old Labrador retriever (FIG. 3B), a 13-year-old Labrador retriever (FIG. 3C), an 8-year-old Australian Shepherd (FIG. 3D), a 13-year-old Tibetan terrier (FIG. 3E), an 8-year-old German Shepherd dog (FIG. 3F), an 8-year-old Rhodesian ridgeback (FIG. 3G), a 13-year-old Pembroke Welsh corgi (FIG. 3H), and a 10-year-old Boxer (FIG. 3I). The bar in FIG. 3A indicates the magnification for all spinal cord cross-sections.

**FIGS. 4A-D -** Skeletal muscle and peripheral nerve histopathology in advanced DM. (FIG. 4A) H&Estained paraffin sections of the gastrocnemius muscle from a 13-year-old DM-affected Pembroke Welsh corgi showed excessive variability in myofiber size with largeandsmall groups of atrophic fibers consistent with denervation. (FIG. 4B) For comparison, a similarly stained gastrocnemius muscle from an age-matched control dog. (FIG. 4C) Toluidine blue stained resin embedded sections of the peroneal nerve from the same Pembroke Welsh corgi showed substantial myelinated fiber loss, endoneurial fibrosis and secondary demyelination. (FIG. 4D) For comparison, a similarly stained peroneal nerve from an age-matched control dog. Bars in the lower right of all figures indicate the magnification.

**FIG. 5 -** Distributions of the ages at sampling of the A/A homozygotes in the affected-breed control group and the ages at onset of clinical signs in the DM-affected dogs.

## DETAILED DESCRIPTION OF ILLUSTRATIVE EMBODIMENTS

### I. The Present Invention

Canine degenerative myelopathy (CDM) is a crippling and fatal neurological disease common in a variety of canine breeds. The disease is chronic and progressive, and the only "treatments" - physical therapy and nutrition - have limited ability to slow the onset of the disease. Ultimately, a dog's back legs become useless, at which point euthanasia may be the only option. If euthanasia is delayed, the disease will ascend to affect the front legs. Progression of the disease is generally slow but unavoidable. As discussed above, the etiology of this disease is unknown, although it is considered that the basis may be genetic. It is therefore of great importance to breeders to be able to identify dogs that carry a genetic risk factor so that they can avoid matings that will produce puppies that are at increased risk of developing CDM when they get older. In addition, a dog's genotype with respect to a genetic risk factor can help confirm or refute a diagnosis of CDM in a dog showing clinical signs of the disease.

The inventors have mapped the risk locus for CDM to region of CFA31 in Pembroke Welsh corgis. Within this chromosomal region, they discovered a missense mutation (E40K) in the canine superoxide dismutase 1 (SOD1) gene, and observed that the K allele is common in several other breeds. Finally, the inventors have demonstrated that homozygosity for the K allele is a major genetic risk factor for CDM. It is of great importance to breeders and owners to be able to identify "at risk" animals, as well as animals that could pass the disease on to future generations. Genetic markers for the high-risk allele will facilitate CDM diagnosis. These markers will also enable breeders to select matings that will produce puppies with greatly reduced risk of developing CDM. Missense mutations in the human SOD1 gene cause amyotrophic lateral sclerosis (ALS), a disease with many clinical and pathological similarities to CDM. Therefore, dogs with CDM are potential animal models for the human disease ALS. DMA markers can help identify dogs that are homozygous for the A allele and can thus be used as an animal model to investigate the underlying disease menchanism or to evaluate potential treatments for ALS or CDM.

### II. Canine Degenerative Myelopathy (CDM)

Canine degenerative myelopathy (also known as chronic degenerative radiculomyelopathy) is a neurological disease prevalent in German shepherds, boxers, Rhodesian Ridgeback, Chesapeake Bay retriever, Pemroke and Cardigan Welsh Corgis, and other pure- and cross-bred dogs. The disease is chronic and progressive, and can result in lameness in the animal, and eventually death.

The disease usually manifests between the ages of seven and fourteen and initially affects the back legs and causes muscle weakness and loss, and lack of coordination. These cause a staggering effect that may appear to be arthritis. The dog may scuff its one or both rear paws when it walks. This scuffing can cause the nails of one foot to be worn down. Eventually the condition may lead to extensive paralysis of the back legs. As the disease progresses the animal starts having considerable difficulties walking, and eventually the back legs become useless, at which point euthanasia may be the only option. If euthanasia is delayed, the clinical signs will progress to affect the front legs. Progression of the disease is generally slow.. The animal could be crippled within a few months, or members of smaller breeds may survive up to 3 years as owners may delay euthanasia because they can easily carry smaller dogs.

Known causes of spinal cord dysfunction should be excluded before accepting the diagnosis of degenerative myelopathy; cervical disc disease (protrusion or rupture) can cause contusions and compression of the spinal cord with all of the signs of degenerative myelopathy. Canine degenerative myelopathy can only be diagnosed by histopathology of the spinal cord. Lesions demonstrate axonal and myelin loss replaced by gliosis in all funciuli of the spinal cord most severe in the dorsal portion of the lateral funiculus of the mid to caudal thoracic spinal cord.

This is a chronic condition that cannot be cured; however, it may be possible to temporarily maintain the dog's quality of life through a proper program of exercise and nutrition. There are no proven effective therapies to alter progression of degenerative myelopathy. Exercise has been recommended to maintain the dog's ability to walk, and physiotherapy may prolong the length of time that the dog remains mobile and increase survival time. Canine hydrotherapy (swimming) may be more useful than walking. Although not approved by the U.S. Food and Drug Administration (FDA), aminocaproic acid (EACA) and n-acetylcysteine (NAC) are thought by some to alter progression.

### III. Superoxide Dismutase

The enzyme superoxide dismutase (SOD) catalyzes the dismutation of superoxide into oxygen and hydrogen peroxide. As such, it is an important antioxidant defense in nearly all cells exposed to oxygen. The SOD-catalysed dismutation of superoxide may be written with the following half-reactions:

$$M^{(n+1)+} - SOD + O_2^- \rightarrow M^{n+} - SOD + O_2$$

$$M^{n+} - SOD + O_2 + 2H^+ \rightarrow M^{(+1)+} - SOD + H_2O_2.$$

where M = Cu (n=1) ; Mn (n=2) ; Fe (n=2) ; Ni (n=2). In this reaction, the oxidation state of the metal cation oscillates between n and n+1.

SODs were prior were known as several metalloproteins with unknown function (for example, CuZnSOD was known as erythrocuprein). Several common forms of SOD exist, cofactored with copper and zinc, or manganese, iron, or nickel.

Chicken liver (and nearly all other) mitochondria, and many bacteria (such as *E. coli*) contain a form with manganese (Mn-SOD). The ligands of the manganese ions are 3 histidine side chains, an aspartate side chain and a water molecule or hydroxy ligand depending on the Mn oxidation state (respectively II and III). *E. coli* and many other bacteria also contain a form of the enzyme with iron (Fe-SOD); some bacteria contain Fe-SOD, others Mn-SOD, and some contain both. The active sites of Mn and Fe superoxide dismutases contain the same type of amino acids side chains. In humans, three forms of superoxide dismutase are present. SOD1 is located in the cytoplasm, SOD2 in the mitochondria and SOD3 is extracellular. The first is a dimer (consists of two units), while the others are tetramers (four subunits). SOD1 and SOD3 contain copper and zinc, while SOD2 has manganese in its reactive centre. The genes are located on human chromosomes 21, 6 and 4, respectively (21q22.1, 6q25.3 and 4p15.3-p15.1). A microtiter plate assay for SOD is available.

### A. SOD1 Function

The cytosols of virtually all eukaryotic cells contain an SOD enzyme with copper and zinc (Cu-Zn-SOD). The Cu-Zn enzyme is a homodimer of molecular weight 32,500. The two subunits are joined primarily by hydrophobic and electrostatic interactions. The ligands of copper and zinc are histidine side chains.

Simply-stated, SOD out competes damaging reactions of superoxide, thus protecting the cell from superoxide toxicity. The reaction of superoxide with non-radicals is spin forbidden. In biological systems, this means its main reactions are with itself (dismutation) or with another biological radical such as nitric oxide (NO). The superoxide anion radical ($O_2^-$) spontaneously dismutes to $O_2$ and hydrogen peroxide ($H_2O_2$) quite rapidly (~$10^5$ $M^{-1}$ $s^{-1}$ at pH 7). SOD is biologically necessary because superoxide reacts even faster with certain targets such as NO radical, which makes peroxynitrite. Similarly, the dismutation rate is second order with respect to initial superoxide concentration. Thus, the half-life of superoxide, although very short at high concentrations (*e.g.*, 0.05 seconds at 0.1 mM) is actually quite long at low concentrations (*e.g.*, 14 hours at 0.1 nM). In contrast, the reaction of superoxide with SOD is first order with respect to superoxide concentration. Moreover, superoxide has the fastest turnover number (reaction rate with its substrate) of any known enzyme (~$10^9$ $M^{-1}s^{-1}$), this reaction being only limited by the frequency of collision between itself and superoxide. That is, the reaction rate is "diffusion limited,"

### B. SOD1 in Disease

Mutations in SOD1 have been linked to familial amyotrophic lateral sclerosis (ALS, a form of motor neuron disease). The other two types have not been linked to any human diseases, however, in mice inactivation of SOD2 causes perinatal lethality and inactivation of SOD1 causes hepatocellular carcinoma. Mutations in SOD1 can cause familial ALS by a mechanism that is presently not understood, but not due to loss of enzymatic activity. Overexpression of SOD1 has been linked to Down's syndrome. Prior to this work, canine SOD1 has not been linked to any particular disease state.

C. Canine SOD1 Protein and cDNA Structure

Wild-type canine cDNA coding sequence:

atggagatgaaggccgtgtgcgtgttgaagggccagggcccggtggagggcaccatccacttcgtgcagaagggaagtg
ggcctgttgtggtatcaggaaccattacagggctgactgaaggcgagcatggattccacgtccatcagtttggagataatacacaaggc
tgtactagtgcaggtcctcactttaatcctctgtccaaaaaacatggtgggccaaaagatcaagagaggcatgttggagacctgggcaat
gtgactgctggcaaggatggcgtggccattgtgtccatagaagattctctgattgcactctcaggagactattccatcattggccgcacca
tggtggtccacgagaaacgagatgacttgggcaaaggtgacaatgaagaaagtacacagacaggaaacgccgggagtcgtttggctt
gtggtgtcattgggatcgccaagtaa (SEQ ID NO:6)

Variant canine cDNA coding sequence:

atggagatgaaggccgtgtgcgtgttgaagggccagggcccggtggagggcaccatccacttcgtgcagaagggaagtgggcctgt
tgtggtatcaggaaccattacagggctgactaaaggcgagcatggattccacgtccatcagtttggagataatacacaaggctgtacta
gtgcaggtcctcactttaatcctctgtccaaaaaacatggtgggccaaaagatcaagagaggcatgttggagacctgggcaatgtgact
gctggcaaggatggcgtggccattgtgtccatagaagattctctgattgcactctcaggagactattccatcattggccgcaccatggtg
gtccacgagaaacgagatgacttgggcaaaggtgacaatgaagaaagtacacagacaggaaacgccgggagtcgtttggcttgtggt
gtcattgggatcgccaagtaa (SEQ ID NO:7)

Predicted wild-type amino acid sequence:

MEMKAVCVLKGQGPVEGTIHFVQKGSGPVVVSGTITGLTEGEHGFHVHQFGDNTQG
CTSAGPHFNPLSKKHGGPKDQERHVGDLGNVTAGKDGVAIVSIEDSLIALSGDYSIIG
RTMVVHEKRDDLGKGDNEESTQTGNAGSRLACGVIGIAK* (SEQ ID NO:8)

Predicted variant amino acid sequence:

MEMKAVCVLKGQGPVEGTIHFVQKGSGPVVVSGTITGLTKGEHGFHVHQFGDNTQG
CTSAGPHFNPLSKKHGGPKDQERHVGDLGNVTAGKDGVAIVSIEDSLIALSGDYSIIG
RTMVVHEKRDDLGKGDNEESTQTGNAGSRLACGVIGIAK* (SEQ ID NO:9)

### IV. Genetic Screening of Canine SOD1

Because the genetic variant is in a coding region of the SOD1 gene and affects the encoded protein, the presence of the E40K polymorphism can be determined from either the sequence of the SOD1 protein, the sequence of the SOD1 nucleic acid or a genetic marker that is in linkage disequilibrium with the E40K polymorphism. As a result, a variety of different methodologies can be employed.

### A. Nucleic Acids

Certain embodiments of the present disclosure concern the analysis of nucleic acids, including the use of amplification primers, oligonucleotide probes, and other nucleic acid elements involved in the analysis of genomic DNA, cDNA or mRNA transcripts. The term "nucleic acid" is well known in the art. A "nucleic acid" as used herein will generally refer to a molecule (*i.e.,* a strand) of DNA or RNA comprised in part by a nucleotide bases. Nucleobases include, for example, naturally-occurring purine or pyrimidine bases found in DNA *(e.g.,* an adenine "A," a guanine "G," a thymine "T" or a cytosine "C") or RNA *(e.g.,* an A, a G, an uracil "U" or a C). The term "nucleic acid" encompass the terms "oligonucleotide" and "polynucleotide," each as a subgenus of the term "nucleic acid." The term "oligonucleotide" refers to a molecule of between about 3 and about 100 nucleobases in length. The term "polynucleotide" refers to at least one molecule of greater than about 100 nucleobases in length. A "gene" refers to coding sequence of a gene product, as well as introns and the promoter of the gene product.

In some embodiments, nucleic acids of the disclosure comprise or are complementary to all or 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 110, 120, 130, 140, 150, 160, 170, 180, 190, 200, 210, 220, 230, 240, 250, 260, 270, 280, 290, 300, 310, 320, 330, 340, 350, 360, 370, 380, 390, 400, 410, 420, 430, 440, 450, 460, 470, 480, 490, 500, 510, 520, 530, 540, 550, 560, 570, 580, 590, 600, 610, 620, 630, 640, 650, 660, 670, 680, 690, 700, 710, 720, 730, 740, 750, 760, 770, 780, 790, 800, 810, 820, 830, 840, 850, 860, 870, 880, 890, 900, 910, 920, 930, 940, 950, 960, 970, 980, 990, 1000, 1100, 1165, 1200, 1300, 1400, 1500 or more contiguous nucleotides, or any range derivable therein, of the canine SOD1 sequence with either a G or A at position 118 of the cDNA sequence of canine SOD1. One of skill in the art knows how to design and use primers and probes for hybridization and amplification, including the limits of homology needed to implement primers and probes.

These definitions generally refer to a single-stranded molecule, but in specific embodiments will also encompass an additional strand that is partially, substantially or fully complementary to the single-stranded molecule. Thus, a nucleic acid may encompass a double-stranded molecule or a triple-stranded molecule that comprises one or more complementary strand(s) or "complement(s)" of a particular sequence comprising a molecule. As used herein, a single-stranded nucleic acid may be denoted by the prefix "ss," a double-stranded nucleic acid by the prefix "ds," and a triple-stranded nucleic acid by the prefix "ts."

In particular aspects, a nucleic acid encodes a protein, polypeptide, or peptide. In certain embodiments, the present disclosure concerns novel compositions comprising at least one proteinaceous molecule. As used herein, a "proteinaceous molecule," "proteinaceous composition," "proteinaceous compound," "proteinaceous chain," or "proteinaceous material" generally refers, but is not limited to, a protein of greater than about 200 amino acids or the full length endogenous sequence translated from a gene; a polypeptide of greater than about 100 amino acids; and/or a peptide of from about 3 to about 100 amino acids. All the "proteinaceous" terms described above may be used interchangeably herein.

**1. Preparation of Nucleic Acids**

A nucleic acid may be made by any technique known to one of ordinary skill in the art, such as for example, chemical synthesis, enzymatic production or biological production. Non-limiting examples of a synthetic nucleic acid *(e.g.,* a synthetic oligonucleotide), include a nucleic acid made by *in vitro* chemical synthesis using phosphotriester, phosphite or phosphoramidite chemistry and solid phase techniques such as described in European Patent 266,032, or via deoxynucleoside H-phosphonate intermediates as described by Froehler *et al., 1986* and U.S. Patent 5,705,629, each incorporated herein by reference. In the methods of the present disclosure one or more oligonucleotide may be used. Various different mechanisms of oligonucleotide synthesis have been disclosed in for example, U.S. Patents 4,659,774, 4,816,571, 5,141,813, 5,264,566, 4,959,463, 5,428,148, 5,554,744, 5,574,146, 5,602,244.

A non-limiting example of an enzymatically produced nucleic acid include one produced by enzymes in amplification reactions such as PCR™ (see for example, U.S. Patent 4,683,202 and U.S. Patent 4,682,195 or the synthesis of an oligonucleotide described in U.S. Patent 5,645,897. A non-limiting example of a biologically produced nucleic acid includes a recombinant nucleic acid produced (*i.e*., replicated) in a living cell, such as a recombinant DNA vector replicated in bacteria (see for example, Sambrook *et al.* 2001)

**2. Purification of Nucleic Acids**

A nucleic acid may be purified on polyacrylamide gels, cesium chloride centrifugation gradients, chromatography columns or by any other means known to one of ordinary skill in the art (see for example, Sambrook *et al.,* 2001) . In some aspects, a nucleic acid is a pharmacologically acceptable nucleic acid. Pharmacologically acceptable compositions are known to those of skill in the art, and are described herein.

In certain aspects, the present disclosure concerns a nucleic acid that is an isolated nucleic acid. As used herein, the term "isolated nucleic acid" refers to a nucleic acid molecule (*e.g*., an RNA or DNA molecule) that has been isolated free of, or is otherwise free of, the bulk of the total genomic and transcribed nucleic acids of one or more cells. In certain embodiments, "isolated nucleic acid" refers to a nucleic acid that has been isolated free of, or is otherwise free of, bulk of cellular components or *in vitro* reaction components such as for example, macromolecules such as lipids or proteins, small biological molecules, and the like.

**3. Nucleic Acid Segments**

In certain embodiments, the nucleic acid is a nucleic acid segment. As used herein, the term "nucleic acid segment," are fragments of a nucleic acid, such as, for a non-limiting example, those that encode only part of a gene locus or a gene sequence. Thus, a "nucleic acid segment" may comprise any part of a gene sequence, including from about 2 nucleotides to the full length gene including promoter regions to the polyadenylation signal and any length that includes all the coding region.

Various nucleic acid segments may be designed based on a particular nucleic acid sequence, and may be of any length. By assigning numeric values to a sequence, for example, the first residue is 1, the second residue is 2, *etc.,* an algorithm defining all nucleic acid segments can be created:

$$n \text{ to } n + y$$

where n is an integer from 1 to the last number of the sequence and y is the length of the nucleic acid segment minus one, where n + y does not exceed the last number of the sequence. Thus, for a 10-mer, the nucleic acid segments correspond to bases 1 to 10, 2 to 11, 3 to 12 ... and so on. For a 15-mer, the nucleic acid segments correspond to bases 1 to 15, 2 to 16, 3 to 17 ... and so on. For a 20-mer, the nucleic segments correspond to bases 1 to 20, 2 to 21, 3 to 22 ... and so on. In certain embodiments, the nucleic acid segment may be a probe or primer. As used herein, a "probe" generally refers to a nucleic acid used in a detection method or composition. As used herein, a "primer" generally refers to a nucleic acid used in an extension or amplification method or composition.

**4. Nucleic Acid Complements**

The present disclosure also encompasses a nucleic acid that is complementary to a nucleic acid. A nucleic acid is "complement(s)" or is "complementary" to another nucleic acid when it is capable of base-pairing with another nucleic acid according to the standard Watson-Crick, Hoogsteen or reverse Hoogsteen binding complementarity rules. As used herein "another nucleic acid" may refer to a separate molecule or a spatial separated sequence of the same molecule. In preferred embodiments, a complement is a hybridization probe or amplification primer for the detection of a nucleic acid polymorphism.

As used herein, the term "complementary" or "complement" also refers to a nucleic acid comprising a sequence of consecutive nucleobases or semiconsecutive nucleobases *(e.g.,* one or more nucleobase moieties are not present in the molecule) capable of hybridizing to another nucleic acid strand or duplex even if less than all the nucleobases do not base pair with a counterpart nucleobase. However, in some diagnostic or detection embodiments, completely complementary nucleic acids are preferred.

**5. Nucleic Acid Detection and Evaluation**

Those in the art will readily recognize that nucleic acid molecules may be double-stranded molecules and that reference to a particular site on one strand refers, as well, to the corresponding site on a complementary strand. Thus, in defining a polymorphic site, reference to an adenine, a thymine (uridine), a cytosine, or a guanine at a particular site on the plus (sense or coding) strand of a nucleic acid molecule is also intended to include the thymine (uridine), adenine, guanine, or cytosine (respectively) at the corresponding site on a minus (antisense or noncoding) strand of a complementary strand of a nucleic acid molecule. Thus, reference may be made to either strand and still comprise the same polymorphic site and an oligonucleotide may be designed to hybridize to either strand. Throughout the text, in identifying a polymorphic site, reference is made to the sense strand, only for the purpose of convenience.

Typically, the nucleic acid mixture is isolated from a biological sample taken from the individual, such as a blood sample or tissue sample using standard techniques such as disclosed in Jones (1963). Suitable tissue samples include whole blood, semen saliva, tears, urine, fecal material, sweat, buccal mucosa, skin and hair. The nucleic acid mixture may be comprised of genomic DNA, mRNA, or cDNA and, in the latter two cases, the biological sample must be obtained from an organ in which the SOD1 gene is expressed. Furthermore it will be understood by the skilled artisan that mRNA or cDNA preparations would not be used to detect polymorphisms located in introns or in 5' and 3' nontranscribed regions. If a SOD1 gene fragment is isolated, it must contain the polymorphic site(s) to be genotyped.

In the genotyping methods used in the present disclosure, the identity of a nucleotide (or nucleotide pair) at a polymorphic site may be determined by amplifying a target region(s) containing the polymorphic site(s) directly from one or both copies of the SOD1 gene present in the individual and the sequence of the amplified region(s) determined by conventional methods. It will be readily appreciated by the skilled artisan that only one nucleotide will be detected at a polymorphic site in individuals who are homozygous at that site, while two different nucleotides will be detected if the individual is heterozygous for that site. The polymorphism may be identified directly, known as positive-type identification, or by inference, referred to as negative-type identification. For example, where a SNP is known to be guanine and cytosine in a reference population, a site may be positively determined to be either guanine or cytosine for an individual homozygous at that site, or both guanine and cytosine, if the individual is heterozygous at that site. Alternatively, the site may be negatively determined to be not guanine (and thus cytosine/cytosine) or not cytosine (and thus guanine/guanine).

The target region(s) may be amplified using any oligonucleotide-directed amplification method, including but not limited to polymerase chain reaction (PCR) (U.S. Patent 4,965,188), ligase chain reaction (LCR) (Barany *et al.,* 1991; WO90/01069), and oligonucleotide ligation assay (OLA) (Landegren *et al.,* 1988). Oligonucleotides useful as primers or probes in such methods should specifically hybridize to a region of the nucleic acid that contains or is adjacent to the polymorphic site. Typically, the oligonucleotides are between 10 and 35 nucleotides in length and preferably, between 15 and 30 nucleotides in length. Most preferably, the oligonucleotides are 20 to 25 nucleotides long. The exact length of the oligonucleotide will depend on many factors that are routinely considered and practiced by the skilled artisan.

Other known nucleic acid amplification procedures may be used to amplify the target region including transcription-based amplification systems (U.S. Patent 5,130,238; EP 329,822; U.S. Patents 5,169,766, WO89/06700) and isothermal methods (Walker *et al.,* 1992).

A polymorphism in the target region may also be assayed before or after amplification using one of several hybridization-based methods known in the art. Typically, allele-specific oligonucleotides are utilized in performing such methods. The allele-specific oligonucleotides may be used as differently labeled probe pairs, with one member of the pair showing a perfect match to one variant of a target sequence and the other member showing a perfect match to a different variant. In some embodiments, more than one polymorphic site may be detected at once using a set of allele-specific oligonucleotides or oligonucleotide pairs.

Hybridization of an allele-specific oligonucleotide to a target polynucleotide may be performed with both entities in solution, or such hybridization may be performed when either the oligonucleotide or the target polynucleotide is covalently or noncovalently affixed to a solid support. Attachment may be mediated, for example, by antibody-antigen interactions, poly-L-Lys, streptavidin or avidin-biotin, salt bridges, hydrophobic interactions, chemical linkages, UV cross-linking baking, etc. Allele-specific oligonucleotides may be synthesized directly on the solid support or attached to the solid support subsequent to synthesis. Solid-supports suitable for use in detection methods of the invention include substrates made of silicon, glass, plastic, paper and the like, which may be formed, for example, into wells (as in 96-well plates), slides, sheets, membranes, fibers, chips, dishes, and beads. The solid support may be treated, coated or derivatized to facilitate the immobilization of the allele-specific oligonucleotide or target nucleic acid.

The genotype for one or more polymorphic sites in the gene of an individual may also be determined by hybridization of one or both copies of the gene, or a fragment thereof, to nucleic acid arrays and subarrays such as described in WO 95/11995. The arrays would contain a battery of allele-specific oligonucleotides representing each of the polymorphic sites to be included in the genotype or haplotype.

The identity of polymorphisms may also be determined using a mismatch detection technique, including but not limited to the RNase protection method using riboprobes (Winter *et al.,* 1985; Meyers *et al.,* 1985) and proteins which recognize nucleotide mismatches, such as the *E. coli* mutS protein (Modrich, 1991). Alternatively, variant alleles can be identified by single strand conformation polymorphism (SSCP) analysis (Orita *et al.,* 1989; Humphries*, et al.,* 1996) or denaturing gradient gel electrophoresis (DGGE) (Wartell *et al.,* 1990; Sheffield *et al.,* 1989).

A polymerase-mediated primer extension method may also be used to identify the polymorphism(s). Several such methods have been described in the patent and scientific literature. Extended primers containing a polymorphism may be detected by mass spectrometry as described in U.S. Patent 5,605,798. Another primer extension method is allele-specific PCR (Ruano *et al.,* 1989; Ruano *et al.,* 1991; WO 93/22456; Turki *et al.,* 1995).

**a. Hybridization**

The use of a probe or primer of between 7, 8, 9, 10, 11, 12, 13, 14, 15, 16, 17, 18, 19, 20, 21, 22, 23, 24, 25, 50, 60, 70, 80, 90, or 100 nucleotides, preferably between 17 and 100 nucleotides in length, or in some aspects of the disclosure up to 1-2 kilobases or more in length, allows the formation of a duplex molecule that is both stable and selective. Molecules having complementary sequences over contiguous stretches greater than 20 bases in length are generally preferred, to increase stability and/or selectivity of the hybrid molecules obtained. One will generally prefer to design nucleic acid molecules for hybridization having one or more complementary sequences of 20 to 30 nucleotides, or even longer where desired. Such fragments may be readily prepared, for example, by directly synthesizing the fragment by chemical means or by introducing selected sequences into recombinant vectors for recombinant production.

Accordingly, the nucleotide sequences of the disclosure may be used for their ability to selectively form duplex molecules with complementary stretches of DNAs and/or RNAs or to provide primers for amplification of DNA or RNA from samples. Depending on the application envisioned, one would desire to employ varying conditions of hybridization to achieve varying degrees of selectivity of the probe or primers for the target sequence.

For applications requiring high selectivity, one will typically desire to employ relatively high stringency conditions to form the hybrids. For example, relatively low salt and/or high temperature conditions, such as provided by about 0.02 M to about 0.10 M NaCl at temperatures of about 50°C to about 70°C. Such high stringency conditions tolerate little, if any, mismatch between the probe or primers and the template or target strand and would be particularly suitable for isolating specific genes or for detecting a specific polymorphism. It is generally appreciated that conditions can be rendered more stringent by the addition of increasing amounts of formamide. For example, under highly stringent conditions, hybridization to filter-bound DNA may be carried out in 0.5 M $NaHPO_4$, 7% sodium dodecyl sulfate (SDS), 1 mM EDTA at 65°C, and washing in 0.1 x SSC/0.1% SDS at 68°C (Ausubel *et al.,* 1989).

Conditions may be rendered less stringent by increasing salt concentration and/or decreasing temperature.

For example, a medium stringency condition could be provided by about 0.1 to 0.25 M NaCl at temperatures of about 37°C to about 55°C, while a low stringency condition could be provided by about 0.15 M to about 0.9 M salt, at temperatures ranging from about 20°C to about 55°C. Under low stringent conditions, such as moderately stringent conditions the washing may be carried out for example in 0.2 x SSC/0.1% SDS at 42°C (Ausubel *et al.,* 1989). Hybridization conditions can be readily manipulated depending on the desired results.

In other embodiments, hybridization may be achieved under conditions of, for example, 50 mM Tris-HCl (pH 8.3), 75 mM KCl, 3 mM $MgCl_2$, 1.0 mM dithiothreitol, at temperatures between approximately 20°C to about 37°C. Other hybridization conditions utilized could include approximately 10 mM Tris-HCl (pH 8.3), 50 mM KC1, 1.5 mM $MgCl_2$, at temperatures ranging from approximately 40°C to about 72°C.

In certain embodiments, it will be advantageous to employ nucleic acids of defined sequences of the present invention in combination with an appropriate means, such as a label, for determining hybridization. A wide variety of appropriate indicator means are known in the art, including fluorescent, radioactive, enzymatic or other ligands, such as avidin/biotin, which are capable of being detected. In preferred embodiments, one may desire to employ a fluorescent label or an enzyme tag such as urease, alkaline phosphatase or peroxidase, instead of radioactive or other environmentally undesirable reagents. In the case of enzyme tags, colorimetric indicator substrates are known that can be employed to provide a detection means that is visibly or spectrophotometrically detectable, to identify specific hybridization with complementary nucleic acid containing samples. In other aspects, a particular nuclease cleavage site may be present and detection of a particular nucleotide sequence can be determined by the presence or absence of nucleic acid cleavage.

In general, it is envisioned that the probes or primers described herein will be useful as reagents in solution hybridization, as in PCR, for detection of expression or genotype of corresponding genes, as well as in embodiments employing a solid phase. In embodiments involving a solid phase, the test DNA (or RNA) is adsorbed or otherwise affixed to a selected matrix or surface. This fixed, single-stranded nucleic acid is then subjected to hybridization with selected probes under desired conditions. The conditions selected will depend on the particular circumstances (depending, for example, on the G+C content, type of target nucleic acid, source of nucleic acid, size of hybridization probe, *etc.*). Optimization of hybridization conditions for the particular application of interest is well known to those of skill in the art. After washing of the hybridized molecules to remove non-specifically bound probe molecules, hybridization is detected, and/or quantified, by determining the amount of bound label. Representative solid phase hybridization methods are disclosed in U.S. Patents 5,843,663, 5,900,481 and 5,919,626. Other methods of hybridization that may be used in the practice of the present invention are disclosed in U.S. Patents 5,849,481, 5,849,486 and 5,851,772. The relevant portions of these and other references identified in this section of the Specification.

**b. Amplification of Nucleic Acids**

Nucleic acids used as a template for amplification may be isolated from cells, tissues or other samples according to standard methodologies (Sambrook *et al.,* 2001). In certain embodiments, analysis is performed on whole cell or tissue homogenates or biological fluid samples with or without substantial purification of the template nucleic acid. The nucleic acid may be genomic DNA or fractionated or whole cell RNA. Where RNA is used, it may be desired to first convert the RNA to a complementary DNA.

The term "primer," as used herein, is meant to encompass any nucleic acid that is capable of priming the synthesis of a nascent nucleic acid in a template-dependent process. Typically, primers are oligonucleotides from ten to twenty and/or thirty base pairs in length, but longer sequences can be employed. Primers may be provided in double-stranded and/or single-stranded form, although the single-stranded form is preferred.

Pairs of primers designed to selectively hybridize to nucleic acids corresponding to the SOD1 gene locus, variants and fragments thereof are contacted with the template nucleic acid under conditions that permit selective hybridization. Depending upon the desired application, high stringency hybridization conditions may be selected that will only allow hybridization to sequences that are completely complementary to the primers. In other embodiments, hybridization may occur under reduced stringency to allow for amplification of nucleic acids that contain one or more mismatches with the primer sequences. Once hybridized, the template-primer complex is contacted with one or more enzymes that facilitate template-dependent nucleic acid synthesis. Multiple rounds of amplification, also referred to as "cycles," are conducted until a sufficient amount of amplification product is produced.

The amplification product may be detected, analyzed or quantified. In certain applications, the detection may be performed by visual means. In certain applications, the detection may involve indirect identification of the product via chemiluminescence, radioactive scintigraphy of incorporated radiolabel or fluorescent label or even via a system using electrical and/or thermal impulse signals (Affymax technology; Bellus, 1994).

A number of template dependent processes are available to amplify the oligonucleotide sequences present in a given template sample. One of the best known amplification methods is the polymerase chain reaction (referred to as PCR™) which is described in detail in U.S. Patents 4,683,195, 4,683,202 and 4,800,159, and in Innis *et al.,* 1988.

Another method for amplification is ligase chain reaction ("LCR"), disclosed in European Application No. 320 308, incorporated herein by reference in its entirety. U.S. Patent 4,883,750 describes a method similar to LCR for binding probe pairs to a target sequence. A method based on PCR™ and oligonucleotide ligase assay (OLA) (described in further detail below), disclosed in U.S. Patent 5,912,148, may also be used.

Alternative methods for amplification of target nucleic acid sequences that may be used in the practice of the present invention are disclosed in U.S. Patents 5,843,650, 5,846,709, 5,846,783, 5,849,546, 5,849,497, 5,849,547, 5,858,652, 5,866,366, 5,916,776, 5,922,574, 5,928,905, 5,928,906, 5,932,451, 5,935,825, 5,939,291 and 5,942,391, Great Britain Application 2 202 328, and in PCT Application PCT/US89/01025. Qbeta Replicase, described in PCT Application PCT/US87/00880, may also be used as an amplification method in the present disclosure.

An isothermal amplification method, in which restriction endonucleases and ligases are used to achieve the amplification of target molecules that contain nucleotide 5'-[alpha-thio]-triphosphates in one strand of a restriction site may also be useful in the amplification of nucleic acids in the present disclosure (Walker *et al.,* 1992). Strand Displacement Amplification (SDA), disclosed in U.S. Patent 5,916,779, is another method of carrying out isothermal amplification of nucleic acids which involves multiple rounds of strand displacement and synthesis, *i.e.,* nick translation

Other nucleic acid amplification procedures include transcription-based amplification systems (TAS), including nucleic acid sequence based amplification (NASBA) and 3SR (Kwoh *et al.,* 1989; PCT Application WO 88/10315, incorporated herein by reference in their entirety). European Application 329 822 disclose a nucleic acid amplification process involving cyclically synthesizing single-stranded RNA ("ssRNA"), ssDNA, and double-stranded DNA (dsDNA), which may be used in accordance with the present disclosure.

PCT Application WO 89/06700 disclose a nucleic acid sequence amplification scheme based on the hybridization of a promoter region/primer sequence to a target single-stranded DNA ("ssDNA") followed by transcription of many RNA copies of the sequence. This scheme is not cyclic, *i.e.,* new templates are not produced from the resultant RNA transcripts. Other amplification methods include "RACE" and "one-sided PCR" (Frohman, 1990; Ohara *et al.,* 1989).

**c. Detection of Nucleic Acids**

Following any amplification, it may be desirable to separate the amplification product from the template and/or the excess primer. In one embodiment, amplification products are separated by agarose, agarose-acrylamide or polyacrylamide gel electrophoresis using standard methods (Sambrook *et al.,* 2001). Separated amplification products may be cut out and eluted from the gel for further manipulation. Using low melting point agarose gels, the separated band may be removed by heating the gel, followed by extraction of the nucleic acid.

Separation of nucleic acids may also be effected by spin columns and/or chromatographic techniques known in art. There are many kinds of chromatography which may be used in the practice of the present invention, including adsorption, partition, ion-exchange, hydroxylapatite, molecular sieve, reverse-phase, column, paper, thin-layer, and gas chromatography as well as HPLC.

In certain embodiments, the amplification products are visualized, with or without separation. A typical visualization method involves staining of a gel with ethidium bromide and visualization of bands under UV light. Alternatively, if the amplification products are integrally labeled with radio- or fluorometrically-labeled nucleotides, the separated amplification products can be exposed to x-ray film or visualized under the appropriate excitatory spectra.

In one embodiment, following separation of amplification products, a labeled nucleic acid probe is brought into contact with the amplified marker sequence. The probe preferably is conjugated to a chromophore but may be radiolabeled. In another embodiment, the probe is conjugated to a binding partner, such as an antibody or biotin, or another binding partner carrying a detectable moiety.

In particular embodiments, detection is by Southern blotting and hybridization with a labeled probe. The techniques involved in Southern blotting are well known to those of skill in the art (see Sambrook *et al.* , 2001). One example of the foregoing is described in U.S. Patent 5,279,721, incorporated by reference herein, which discloses an apparatus and method for the automated electrophoresis and transfer of nucleic acids. The apparatus permits electrophoresis and blotting without external manipulation of the gel and is ideally suited to carrying out methods according to the present disclosure

Other methods of nucleic acid detection that may be used in the practice of the instant invention are disclosed in U.S. Patents 5,840,873, 5,843,640, 5,843,651, 5,846,708, 5,846,717, 5,846,726, 5,846,729, 5,849,487, 5,853,990, 5,853,992, 5,853,993, 5,856,092, 5,861,244, 5,863,732, 5,863,753, 5,866,331, 5,905,024, 5,910,407, 5,912,124, 5,912,145, 5,919,630, 5,925,517, 5,928,862, 5,928,869, 5,929,227, 5,932,413 and 5,935,791.

**d. Other Assays**

Other methods for genetic screening may be used, for example, to detect mutations in genomic DNA, cDNA and/or RNA samples. Methods used to detect point mutations include denaturing gradient gel electrophoresis (DGGE), restriction fragment length polymorphism analysis (RFLP), chemical or enzymatic cleavage methods, direct sequencing of target regions amplified by PCR™ (see above), single-strand conformation polymorphism analysis (SSCP) and other methods well known in the art.

One method of screening for point mutations is based on RNase cleavage of base pair mismatches in RNA/DNA or RNA/RNA heteroduplexes. As used herein, the term "mismatch" is defined as a region of one or more unpaired or mispaired nucleotides in a double-stranded RNA/RNA, RNA/DNA or DNA/DNA molecule. This definition thus includes mismatches due to insertion/deletion mutations, as well as single or multiple base point mutations.

U.S. Patent 4,946,773 describes an RNase A mismatch cleavage assay that involves annealing single-stranded DNA or RNA test samples to an RNA probe, and subsequent treatment of the nucleic acid duplexes with RNase A. For the detection of mismatches, the single-stranded products of the RNase A treatment, electrophoretically separated according to size, are compared to similarly treated control duplexes. Samples containing smaller fragments (cleavage products) not seen in the control duplex are scored as positive.

Other investigators have described the use of RNase I in mismatch assays. The use of RNase I for mismatch detection is described in literature from Promega Biotech. Promega markets a kit containing RNase I that is reported to cleave three out of four known mismatches. Others have described using the MutS protein or other DNA-repair enzymes for detection of single-base mismatches.

Alternative methods for detection of deletion, insertion or substitution mutations that may be used in the practice of the present disclosure are disclosed in U.S. Patents 5,849,483, 5,851,770, 5,866,337, 5,925,525 and 5,928,870.

**e. Specific Examples of Polymorphism Nucleic Acid Screening Methods**

Spontaneous mutations that arise during the course of evolution in the genomes of organisms are often not immediately transmitted throughout all of the members of the species, thereby creating polymorphic alleles that co-exist in the species populations. Often polymorphisms are the cause of genetic diseases. Several classes of polymorphisms have been identified. For example, variable number tandem repeat polymorphisms (VNTRs), arise from spontaneous tandem duplications of di- or trinucleotide repeated motifs of nucleotides. If such variations alter the lengths of DNA fragments generated by restriction endonuclease cleavage, the variations are referred to as restriction fragment length polymorphisms (RFLPs). RFLPs are been widely used in human and animal genetic analyses.

Another class of polymorphisms is generated by the replacement of a single nucleotide. Such single nucleotide polymorphisms (SNPs) can result in changes in a restriction endonuclease site. Thus, SNPs are sometimes detectable by restriction fragment length analysis. SNPs are the most common genetic variations and occur once every 100 to 300 bases and several SNP mutations have been found that affect a single nucleotide in a protein-encoding gene in a manner sufficient to actually cause a genetic disease. SNP diseases are exemplified by hemophilia, sickle-cell anemia, hereditary hemochromatosis, late-onset alzheimer disease *etc.*

Several methods have been developed to screen polymorphisms and some examples are listed below. The reference of Kwok and Chen (2003) and Kwok (2001) provide overviews of some of these methods; both of these references are specifically incorporated by reference.

SNPs relating to SOD1 can be characterized by the use of any of these methods or suitable modification thereof. Such methods include the direct or indirect sequencing of the site, the use of restriction enzymes where the respective alleles of the site create or destroy a restriction site, the use of allele-specific hybridization probes, the use of antibodies that are specific for the proteins encoded by the different alleles of the polymorphism, or any other biochemical interpretation.

**i. DNA Sequencing**

The most commonly used method of characterizing a polymorphism is direct DNA sequencing of the genetic locus that flanks and includes the polymorphism. Such analysis can be accomplished using either the "dideoxy-mediated chain termination method," also known as the "Sanger Method" (Sanger *et al.,* 1975) or the "chemical degradation method," also known as the "Maxam-Gilbert method" (Maxam *et al.,* 1977). Sequencing in combination with genomic sequence-specific amplification technologies, such as the polymerase chain reaction may be utilized to facilitate the recovery of the desired genes (Mullis *et al.,* 1986; European Patent Application 50,424; European Patent Application. 84,796, European Patent Application 258,017, European Patent Application. 237,362; European Patent Application. 201,184; U.S. Patents 4,683,202; 4,582,788; and 4.683,194).

**ii. Exonuclease Resistance**

Other methods that can be employed to determine the identity of a nucleotide present at a polymorphic site utilize a specialized exonuclease-resistant nucleotide derivative (U.S. Patent. 4,656,127). A primer complementary to an allelic sequence immediately 3'-to the polymorphic site is hybridized to the DNA under investigation. If the polymorphic site on the DNA contains a nucleotide that is complementary to the particular exonucleotide-resistant nucleotide derivative present, then that derivative will be incorporated by a polymerase onto the end of the hybridized primer. Such incorporation makes the primer resistant to exonuclease cleavage and thereby permits its detection. As the identity of the exonucleotide-resistant derivative is known one can determine the specific nucleotide present in the polymorphic site of the DNA.

**iii. Microsequencing Methods**

Several other primer-guided nucleotide incorporation procedures for assaying polymorphic sites in DNA have been described (Komher *et al.,* 1989; Sokolov, 1990; Syvanen 1990; Kuppuswamy *et al.,* 1991; Prezant *et al.,* 1992; Ugozzoll *et al.,* 1992; Nyren *et al.,* 1993). These methods rely on the incorporation of labeled deoxynucleotides to discriminate between bases at a polymorphic site. As the signal is proportional to the number of deoxynucleotides incorporated, polymorphisms that occur in runs of the same nucleotide result in a signal that is proportional to the length of the run (Syvanen *et al.*,1990).

**iv. Extension in Solution**

French Patent 2,650,840 and PCT Application WO91/02087 discuss a solution-based method for determining the identity of the nucleotide of a polymorphic site. According to these methods, a primer complementary to allelic sequences immediately 3'-to a polymorphic site is used. The identity of the nucleotide of that site is determined using labeled dideoxynucleotide derivatives which are incorporated at the end of the primer if complementary to the nucleotide of the polymorphic site.

**v. Genetic Bit Analysis or Solid-Phase Extension**

PCT Application WO92/15712 describes a method that uses mixtures of labeled terminators and a primer that is complementary to the sequence 3' to a polymorphic site. The labeled terminator that is incorporated is complementary to the nucleotide present in the polymorphic site of the target molecule being evaluated and is thus identified. Here the primer or the target molecule is immobilized to a solid phase.

**vi. Oligonucleotide Ligation Assay (OLA)**

This is another solid phase method that uses different methodology (Landegren *et al.,* 1988). Two oligonucleotides, capable of hybridizing to abutting sequences of a single strand of a target DNA are used. One of these oligonucleotides is biotinylated while the other is detectably labeled. If the precise complementary sequence is found in a target molecule, the oligonucleotides will hybridize such that their termini abut, and create a ligation substrate. Ligation permits the recovery of the labeled oligonucleotide by using avidin. Other nucleic acid detection assays, based on this method, combined with PCR have also been described (Nickerson *et al.,* 1990). Here, PCR is used to achieve the exponential amplification of target DNA, which is then detected using the OLA.

**vii. Ligase/Polymerase-Mediated Genetic Bit Analysis**

U.S. Patent 5,952,174 describes a method that also involves two primers capable of hybridizing to abutting sequences of a target molecule. The hybridized product is formed on a solid support to which the target is immobilized. Here the hybridization occurs such that the primers are separated from one another by a space of a single nucleotide. Incubating this hybridized product in the presence of a polymerase, a ligase, and a nucleoside triphosphate mixture containing at least one deoxynucleoside triphosphate allows the ligation of any pair of abutting hybridized oligonucleotides. Addition of a ligase results in two events required to generate a signal, extension and ligation. This provides a higher specificity and lower "noise" than methods using either extension or ligation alone and unlike the polymerase-based assays, this method enhances the specificity of the polymerase step by combining it with a second hybridization and a ligation step for a signal to be attached to the solid phase.

**viii. Invasive Cleavage Reactions**

Invasive cleavage reactions can be used to evaluate cellular DNA for a particular polymorphism. A technology called INVADER® employs such reactions (*e.g.,* de Arruda *et al.,* 2002; Stevens *et al.,* 2003). Generally, there are three nucleic acid molecules: 1) an oligonucleotide upstream of the target site ("upstream oligo"), 2) a probe oligonucleotide covering the target site ("probe"), and 3) a single-stranded DNA with the the target site ("target"). The upstream oligo and probe do not overlap but they contain contiguous sequences. The probe contains a donor fluorophore, such as fluoroscein, and an acceptor dye, such as Dabcyl. The nucleotide at the 3' terminal end of the upstream oligo overlaps ("invades") the first base pair of a probe-target duplex. Then the probe is cleaved by a structure-specific 5' nuclease causing separation of the fluorophore/quencher pair, which increases the amount of fluorescence that can be detected. *See* Lu *et al.* (2004). In some cases, the assay is conducted on a solid-surface or in an array format.

**ix. Other Methods To Detect SNPs**

Several other specific methods for polymorphism detection and identification are presented below and may be used as such or with suitable modifications in conjunction with identifying polymorphisms of the SOD1 gene in the present invention. Several other methods are also described on the SNP web site of the NCBI on the World Wide Web at ncbi.nlm.nih.gov/SNP.

In a particular embodiment, extended haplotypes may be determined at any given locus in a population, which allows one to identify exactly which SNPs will be redundant and which will be essential in association studies. The latter is referred to as 'haplotype tag SNPs (htSNPs)', markers that capture the haplotypes of a gene or a region of linkage disequilibrium. See Johnson *et al.* (2001) and Ke and Cardon (2003), each of which is incorporated herein by reference, for exemplary methods.

The VDA-assay utilizes PCR amplification of genomic segments by long PCR methods using TaKaRa LA Taq reagents and other standard reaction conditions. The long amplification can amplify DNA sizes of about 2,000-12,000 bp. Hybridization of products to variant detector array (VDA) can be performed by a Affymetrix High Throughput Screening Center and analyzed with computerized software.

A method called Chip Assay uses PCR amplification of genomic segments by standard or long PCR protocols. Hybridization products are analyzed by VDA, Halushka *et al.* (1999), incorporated herein by reference. SNPs are generally classified as "certain" or "likely" based on computer analysis of hybridization patterns. By comparison to alternative detection methods such as nucleotide sequencing, "certain" SNPs have been confirmed 100% of the time; and "likely" SNPs have been confirmed 73% of the time by this method.

Other methods simply involve PCR amplification following digestion with the relevant restriction enzyme. Yet others involve sequencing of purified PCR products from known genomic regions.

In yet another method, individual exons or overlapping fragments of large exons are PCR-amplified. Primers are designed from published or database sequences and PCR-amplification of genomic DNA is performed using the following conditions: 200 ng DNA template, 0.5 $\mu$M each primer, 80 $\mu$M each of dCTP, dATP, dTTP and dGTP, 5% formamide, 1.5 mM $MgCl_2$, 0.5 U of Taq polymerase and 0.1 volume of the Taq buffer. Thermal cycling is performed and resulting PCR-products are analyzed by PCR-single strand conformation polymorphism (PCR-SSCP) analysis, under a variety of conditions, *e.g,* 5 or 10% polyacrylamide gel with 15% urea, with or without 5% glycerol. Electrophoresis is performed overnight. PCR-products that show mobility shifts are reamplified and sequenced to identify nucleotide variation.

In a method called CGAP-GAI (DEMIGLACE), sequence and alignment data (from a PHRAP.ace file), quality scores for the sequence base calls (from PHRED quality files), distance information (from PHYLIP dnadist and neighbour programs) and base-calling data (from PHRED '-d' switch) are loaded into memory. Sequences are aligned and examined for each vertical chunk ('slice') of the resulting assembly for disagreement. Any such slice is considered a candidate SNP (DEMIGLACE). A number of filters are used by DEMIGLACE to eliminate slices that are not likely to represent true polymorphisms. These include filters that: (i) exclude sequences in any given slice from SNP consideration where neighboring sequence quality scores drop 40% or more; (ii) exclude calls in which peak amplitude is below the fifteenth percentile of all base calls for that nucleotide type; (iii) disqualify regions of a sequence having a high number of disagreements with the consensus from participating in SNP calculations; (iv) removed from consideration any base call with an alternative call in which the peak takes up 25% or more of the area of the called peak; (v) exclude variations that occur in only one read direction. PHRED quality scores were converted into probability-of-error values for each nucleotide in the slice. Standard Baysian methods are used to calculate the posterior probability that there is evidence of nucleotide heterogeneity at a given location.

In a method called CU-RDF (RESEQ), PCR amplification is performed from DNA isolated from blood using specific primers for each SNP, and after typical cleanup protocols to remove unused primers and free nucleotides, direct sequencing using the same or nested primers.

In a method called DEBNICK (METHOD-B), a comparative analysis of clustered EST sequences is performed and confirmed by fluorescent-based DNA sequencing. In a related method, called DEBNICK (METHOD-C), comparative analysis of clustered EST sequences with phred quality > 20 at the site of the mismatch, average phred quality >= 20 over 5 bases 5'-FLANK and 3' to the SNP, no mismatches in 5 bases 5' and 3' to the SNP, at least two occurrences of each allele is performed and confirmed by examining traces.

In a method identified by ERO (RESEQ), new primers sets are designed for electronically published STSs and used to amplify DNA from 10 different mouse strains. The amplification product from each strain is then gel purified and sequenced using a standard dideoxy, cycle sequencing technique with $^{33}P$-labeled terminators. All the ddATP terminated reactions are then loaded in adjacent lanes of a sequencing gel followed by all of the ddGTP reactions and so on. SNPs are identified by visually scanning the radiographs.

In another method identified as ERO (RESEQ-HT), new primers sets are designed for electronically published murine DNA sequences and used to amplify DNA from 10 different mouse strains. The amplification product from each strain is prepared for sequencing by treating with Exonuclease I and Shrimp Alkaline Phosphatase. Sequencing is performed using ABI Prism Big Dye Terminator Ready Reaction Kit (Perkin-Elmer) and sequence samples are run on the 3700 DNA Analyzer (96 Capillary Sequencer).

FGU-CBT (SCA2-SNP) identifies a method where the region containing the SNP was PCR amplified using the primers SCA2-FP3 and SCA2-RP3. Approximately 100 ng of genomic DNA is amplified in a 50 ml reaction volume containing a final concentration of 5 mM Tris, 25 mM KC1, 0.75 mM $MgCl_2$, 0.05% gelatin, 20 pmol of each primer and 0.5U of Taq DNA polymerase. Samples are denatured, annealed and extended and the PCR product is purified from a band cut out of the agarose gel using, for example, the QIAquick gel extraction kit (Qiagen) and is sequenced using dye terminator chemistry on an ABI Prism 377 automated DNA sequencer with the PCR primers.

In a method identified as JBLACK (SEQ/RESTRICT), two independent PCR reactions are performed with genomic DNA. Products from the first reaction are analyzed by sequencing, indicating a unique FspI restriction site. The mutation is confirmed in the product of the second PCR reaction by digesting with Fsp I.

In a method described as KWOK(1), SNPs are identified by comparing high quality genomic sequence data from four randomly chosen individuals by direct DNA sequencing of PCR products with dye-terminator chemistry (see Kwok *et al.,* 1996). In a related method identified as KWOK(2) SNPs are identified by comparing high quality genomic sequence data from overlapping large-insert clones such as bacterial artificial chromosomes (BACs) or P1-based artificial chromosomes (PACs). An STS containing this SNP is then developed and the existence of the SNP in various populations is confirmed by pooled DNA sequencing (see Taillon-Miller *et al.,* 1998). In another similar method called KWOK(3), SNPs are identified by comparing high quality genomic sequence data from overlapping large-insert clones BACs or PACs. The SNPs found by this approach represent DNA sequence variations between the two donor chromosomes but the allele frequencies in the general population have not yet been determined. In method KWOK(5), SNPs are identified by comparing high quality genomic sequence data from a homozygous DNA sample and one or more pooled DNA samples by direct DNA sequencing of PCR products with dye-terminator chemistry. The STSs used are developed from sequence data found in publicly available databases. Specifically, these STSs are amplified by PCR against a complete hydatidiform mole (CHM) that has been shown to be homozygous at all loci and a pool of DNA samples from 80 CEPH parents (see Kwok *et al.,* 1994).

In another such method, KWOK (OverlapSnpDetectionWithPolyBayes), SNPs are discovered by automated computer analysis of overlapping regions of large-insert human genomic clone sequences. For data acquisition, clone sequences are obtained directly from large-scale sequencing centers. This is necessary because base quality sequences are not present/available through GenBank. Raw data processing involves analyzed of clone sequences and accompanying base quality information for consistency. Finished ('base perfect', error rate lower than 1 in 10,000 bp) sequences with no associated base quality sequences are assigned a uniform base quality value of 40 (1 in 10,000 bp error rate). Draft sequences without base quality values are rejected. Processed sequences are entered into a local database. A version of each sequence with known human repeats masked is also stored. Repeat masking is performed with the program "MASKERAID." Overlap detection: Putative overlaps are detected with the program "WUBLAST." Several filtering steps followed in order to eliminate false overlap detection results, *i.e.* similarities between a pair of clone sequences that arise due to sequence duplication as opposed to true overlap. Total length of overlap, overall percent similarity, number of sequence differences between nucleotides with high base quality value "high-quality mismatches." Results are also compared to results of restriction fragment mapping of genomic clones at Washington University Genome Sequencing Center, finisher's reports on overlaps, and results of the sequence contig building effort at the NCBI. SNP detection: Overlapping pairs of clone sequence are analyzed for candidate SNP sites with the 'POLYBAYES' SNP detection software. Sequence differences between the pair of sequences are scored for the probability of representing true sequence variation as opposed to sequencing error. This process requires the presence of base quality values for both sequences. High-scoring candidates are extracted. The search is restricted to substitution-type single base pair variations. Confidence score of candidate SNP is computed by the POLYBAYES software.

In method identified by KWOK (TaqMan assay), the TaqMan assay is used to determine genotypes for 90 random individuals. In method identified by KYUGEN(Q1), DNA samples of indicated populations are pooled and analyzed by PLACE-SSCP. Peak heights of each allele in the pooled analysis are corrected by those in a heterozygote, and are subsequently used for calculation of allele frequencies. Allele frequencies higher than 10% are reliably quantified by this method. Allele frequency = 0 (zero) means that the allele was found among individuals, but the corresponding peak is not seen in the examination of pool. Allele frequency = 0-0.1 indicates that minor alleles are detected in the pool but the peaks are too low to reliably quantify.

In yet another method identified as KYUGEN (Method1), PCR products are post-labeled with fluorescent dyes and analyzed by an automated capillary electrophoresis system under SSCP conditions (PLACE-SSCP). Four or more individual DNAs are analyzed with or without two pooled DNA (Japanese pool and CEPH parents pool) in a series of experiments. Alleles are identified by visual inspection. Individual DNAs with different genotypes are sequenced and SNPs identified. Allele frequencies are estimated from peak heights in the pooled samples after correction of signal bias using peak heights in heterozygotes. For the PCR primers are tagged to have 5'-ATT or 5'-GTT at their ends for post-labeling of both strands. Samples of DNA (10 ng/ul) are amplified in reaction mixtures containing the buffer (10 mM Tris-HC1, pH 8.3 or 9.3, 50 mM KC1, 2.0 mM $MgCl_2$), 0.25 $\mu$M of each primer, 200$\mu$M of each dNTP, and 0.025 units/$\mu$l of Taq DNA polymerase premixed with anti-Taq antibody. The two strands of PCR products are differentially labeled with nucleotides modified with R110 and R6G by an exchange reaction of Klenow fragment of DNA polymerase I. The reaction is stopped by adding EDTA, and unincorporated nucleotides are dephosphorylated by adding calf intestinal alkaline phosphatase. For the SSCP: an aliquot of fluorescently labeled PCR products and TAMRA-labeled internal markers are added to deionized formamide, and denatured. Electrophoresis is performed in a capillary using an ABI Prism 310 Genetic Analyzer. Genescan softwares (P-E Biosystems) are used for data collection and data processing. DNA of individuals (two to eleven) including those who showed different genotypes on SSCP are subjected for direct sequencing using big-dye terminator chemistry, on ABI Prism 310 sequencers. Multiple sequence trace files obtained from ABI Prism 310 are processed and aligned by Phred/Phrap and viewed using Consed viewer. SNPs are identified by PolyPhred software and visual inspection.

In yet another method identified as KYUGEN (Method2), individuals with different genotypes are searched by denaturing HPLC (DHPLC) or PLACE-SSCP (Inazuka *et al.,* 1997) and their sequences are determined to identify SNPs. PCR is performed with primers tagged with 5'-ATT or 5'-GTT at their ends for post-labeling of both strands. DHPLC analysis is carried out using the WAVE DNA fragment analysis system (Transgenomic). PCR products are injected into DNASep column, and separated under the conditions determined using WAVEMaker program (Transgenomic). The two strands of PCR products that are differentially labeled with nucleotides modified with R110 and R6G by an exchange reaction of Klenow fragment of DNA polymerase I. The reaction is stopped by adding EDTA, and unincorporated nucleotides are dephosphorylated by adding calf intestinal alkaline phosphatase. SSCP followed by electrophoresis is performed in a capillary using an ABI Prism 310 Genetic Analyzer. Genescan softwares (P-E Biosystems). DNA of individuals including those who showed different genotypes on DHPLC or SSCP are subjected for direct sequencing using big-dye terminator chemistry, on ABI Prism 310 sequencer. Multiple sequence trace files obtained from ABI Prism 310 are processed and aligned by Phred/Phrap and viewed using Consed viewer. SNPs are identified by PolyPhred software and visual inspection. Trace chromatogram data of EST sequences in Unigene are processed with PHRED. To identify likely SNPs, single base mismatches are reported from multiple sequence alignments produced by the programs PHRAP, BRO and POA for each Unigene cluster. BRO corrected possible misreported EST orientations, while POA identified and analyzed non-linear alignment structures indicative of gene mixing/chimeras that might produce spurious SNPs. Bayesian inference is used to weigh evidence for true polymorphism versus sequencing error, misalignment or ambiguity, misclustering or chimeric EST sequences, assessing data such as raw chromatogram height, sharpness, overlap and spacing; sequencing error rates; context-sensitivity; cDNA library origin, etc.

In method identified as MARSHFIELD (Method-B), overlapping human DNA sequences which contained putative insertion/deletion polymorphisms are identified through searches of public databases. PCR primers which flanked each polymorphic site are selected from the consensus sequences. Primers are used to amplify individual or pooled human genomic DNA. Resulting PCR products are resolved on a denaturing polyacrylamide gel and a PhosphorImager is used to estimate allele frequencies from DNA pools.

**f. Linkage Disequilibrium**

Polymorphisms in linkage disequilibrium with another polymorphism in which identification of one polymorphism is predictive of the identity of the linked polymorphism. "Linkage disequilibrium" ("LD" as used herein, though also referred to as "LED" in the art) refers to a situation where a particular combination of alleles (*i.e.,* a variant form of a given gene) or polymorphisms at two loci appears more frequently than would be expected by chance. "Significant" as used in respect to linkage disequilibrium, as determined by one of skill in the art, is contemplated to be a statistical p or $\alpha$ value that may be 0.25 or 0.1 and may be 0.1, 0.05. 0.001, 0.00001 or less. The polymorphism at position 40 SOD1 protein may be determined by evaluating the nucleic acid sequence of a polymorphism in linkage disequilibrium with the residue 40 polymorphism. The invention may be implemented in this manner with respect to one or more polymorphisms so as to allow haplotype analysis. "Haplotype" is used according to its plain and ordinary meaning to one skilled in the art. It refers to a collective genotype of two or more alleles or polymorphisms along one of the homologous chromosomes.

### B. Evaluating the Protein

Alternatively, polymorphic variation can be determined by any method that detects an amino acid variation. The invention should not be limited by any particular method for achieving this. For example, a sample of fluid or tissue may be obtained from an individual and the amino acid at position 40 of the SOD1 protein is determined. Such detection can be by various methods including antibody based assays, (Western blots, ELISA) or amino acid analysis (high pressure liquid chromatography or mass spectroscopy) could be used that would detect whether the protein has Arg or Gly.

Therefore, in certain embodiments, the present invention concerns compositions comprising at least one proteinaceous molecule, such as a SOD1 protein or an protein that binds SOD1 protein, such as an antibody. As used herein, a "proteinaceous molecule," "proteinaceous composition," "proteinaceous compound," "proteinaceous chain" or "proteinaceous material" generally refers, but is not limited to, a protein of greater than about 200 amino acids or the full length endogenous sequence translated from a gene; a polypeptide of greater than about 100 amino acids; and/or a peptide of from about 3 to about 100 amino acids. All the "proteinaceous" terms described above may be used interchangeably herein.

Proteinaceous compositions may be made by any technique known to those of skill in the art, including the expression of proteins, polypeptides or peptides through standard molecular biological techniques, the isolation of proteinaceous compounds from natural sources, or the chemical synthesis of proteinaceous materials. The nucleotide and protein, polypeptide and peptide sequences for various genes have been previously disclosed, and may be found at computerized databases known to those of ordinary skill in the art. One such database is the National Center for Biotechnology Information's Genbank and GenPept databases (the world wide web at ncbi.nlm.nih.gov/). The coding regions for these known genes may be amplified and/or expressed using the techniques disclosed herein or as would be know to those of ordinary skill in the art. Alternatively, various commercial preparations of proteins, polypeptides and peptides are known to those of skill in the art.

#### 1. Protein Purification

It may be desirable to purify SOD1 from a sample or purify a protein that binds SOD1, such as an antibody. Such techniques are widely employed and the invention is not intended to be limited with respect to protein purification. Protein purification techniques are well known to those of skill in the art. These techniques involve, at one level, the crude fractionation of the cellular milieu to polypeptide and non-polypeptide fractions. Having separated the polypeptide from other proteins, the polypeptide of interest may be further purified using chromatographic and electrophoretic techniques to achieve partial or complete purification (or purification to homogeneity). Analytical methods particularly suited to the preparation of a pure peptide are ion-exchange chromatography, exclusion chromatography; polyacrylamide gel electrophoresis; isoelectric focusing. A particularly efficient method of purifying peptides is fast protein liquid chromatography or even HPLC.

Certain aspects of the present invention may concern the purification, and in particular embodiments, the substantial purification, of an encoded protein or peptide. The term "purified protein or peptide" as used herein, is intended to refer to a composition, isolatable from other components, wherein the protein or peptide is purified to any degree relative to its naturally-obtainable state. A purified protein or peptide therefore also refers to a protein or peptide, free from the environment in which it may naturally occur.

Generally, "purified" will refer to a protein or peptide composition that has been subjected to fractionation to remove various other components, and which composition substantially retains its expressed biological activity. Where the term "substantially purified" is used, this designation will refer to a composition in which the protein or peptide forms the major component of the composition, such as constituting about 50%, about 60%, about 70%, about 80%, about 90%, about 95% or more of the proteins in the composition.

Various methods for quantifying the degree of purification of the protein or peptide will be known to those of skill in the art in light of the present disclosure. These include, for example, determining the specific activity of an active fraction, or assessing the amount of polypeptides within a fraction by SDS/PAGE analysis. A preferred method for assessing the purity of a fraction is to calculate the specific activity of the fraction, to compare it to the specific activity of the initial extract, and to thus calculate the degree of purity, herein assessed by a "-fold purification number." The actual units used to represent the amount of activity will, of course, be dependent upon the particular assay technique chosen to follow the purification and whether or not the expressed protein or peptide exhibits a detectable activity.

A variety of techniques suitable for use in protein purification will be well known to those of skill in the art. These include, for example, precipitation with ammonium sulfate, PEG, antibodies and the like or by heat denaturation, followed by centrifugation; chromatography steps such as ion exchange, gel filtration, reverse phase, hydroxylapatite and affinity chromatography; isoelectric focusing; gel electrophoresis; and combinations of such and other techniques. As is generally known in the art, it is believed that the order of conducting the various purification steps may be changed, or that certain steps may be omitted, and still result in a suitable method for the preparation of a substantially purified protein or peptide.

There is no general requirement that the protein or peptide always be provided in their most purified state. Indeed, it is contemplated that less substantially purified products will have utility in certain embodiments. Partial purification may be accomplished by using fewer purification steps in combination, or by utilizing different forms of the same general purification scheme. For example, it is appreciated that a cation-exchange column chromatography performed utilizing an HPLC apparatus will generally result in a greater "-fold" purification than the same technique utilizing a low pressure chromatography system. Methods exhibiting a lower degree of relative purification may have advantages in total recovery of protein product, or in maintaining the activity of an expressed protein.

It is known that the migration of a polypeptide can vary, sometimes significantly, with different conditions of SDS/PAGE (Capaldi *et al.,* 1977). It will therefore be appreciated that under differing electrophoresis conditions, the apparent molecular weights of purified or partially purified expression products may vary.

High Performance Liquid Chromatography (HPLC) is characterized by a very rapid separation with extraordinary resolution of peaks. This is achieved by the use of very fine particles and high pressure to maintain an adequate flow rate. Separation can be accomplished in a matter of minutes, or at most an hour. Moreover, only a very small volume of the sample is needed because the particles are so small and close-packed that the void volume is a very small fraction of the bed volume. Also, the concentration of the sample need not be very great because the bands are so narrow that there is very little dilution of the sample.

Gel chromatography, or molecular sieve chromatography, is a special type of partition chromatography that is based on molecular size. The theory behind gel chromatography is that the column, which is prepared with tiny particles of an inert substance that contain small pores, separates larger molecules from smaller molecules as they pass through or around the pores, depending on their size. As long as the material of which the particles are made does not adsorb the molecules, the sole factor determining rate of flow is the size. Hence, molecules are eluted from the column in decreasing size, so long as the shape is relatively constant. Gel chromatography is unsurpassed for separating molecules of different size because separation is independent of all other factors such as pH, ionic strength, temperature, etc. There also is virtually no adsorption, less zone spreading and the elution volume is related in a simple matter to molecular weight.

Affinity Chromatography is a chromatographic procedure that relies on the specific affinity between a substance to be isolated and a molecule that it can specifically bind to. This is a receptor-ligand type interaction. The column material is synthesized by covalently coupling one of the binding partners to an insoluble matrix. The column material is then able to specifically adsorb the substance from the solution. Elution occurs by changing the conditions to those in which binding will not occur (*e.g*., alter pH, ionic strength, and temperature).

A particular type of affinity chromatography useful in the purification of carbohydrate containing compounds is lectin affinity chromatography. Lectins are a class of substances that bind to a variety of polysaccharides and glycoproteins. Lectins are usually coupled to agarose by cyanogen bromide. Conconavalin A coupled to Sepharose was the first material of this sort to be used and has been widely used in the isolation of polysaccharides and glycoproteins other lectins that have been include lentil lectin, wheat germ agglutinin which has been useful in the purification of N-acetyl glucosaminyl residues and *Helix pomatia* lectin. Lectins themselves are purified using affinity chromatography with carbohydrate ligands. Lactose has been used to purify lectins from castor bean and peanuts; maltose has been useful in extracting lectins from lentils and jack bean; N-acetyl-D galactosamine is used for purifying lectins from soybean; N-acetyl glucosaminyl binds to lectins from wheat germ; D-galactosamine has been used in obtaining lectins from clams and L-fucose will bind to lectins from lotus.

The matrix should be a substance that itself does not adsorb molecules to any significant extent and that has a broad range of chemical, physical and thermal stability. The ligand should be coupled in such a way as to not affect its binding properties. The ligand also should provide relatively tight binding. And it should be possible to elute the substance without destroying the sample or the ligand. One of the most common forms of affinity chromatography is immunoaffinity chromatography. The generation of antibodies that would be suitable for use in accord with the present invention is discussed below.

### 2. Antibodies

Another embodiment of the present disclosure are antibodies, in some cases, a human monoclonal antibody immunoreactive with the polypeptide sequence of canine SOD1. It is understood that antibodies can be used for detecting SOD1, particularly a SOD1 that is the result of a particular polymorphism. It is contemplated that antibodies particularly useful in the context of the present invention are those that differentially bind a SOD1 protein with either an E or K residue and position 40 so as to distinguish between the two populations.

As used herein, the term "antibody" is intended to refer broadly to any immunologic binding agent such as IgG, IgM, IgA, IgD and IgE. Generally, IgG and/or IgM are preferred because they are the most common antibodies in the physiological situation and because they are most easily made in a laboratory setting.

The term "antibody" is used to refer to any antibody-like molecule that has an antigen binding region, and includes antibody fragments such as Fab', Fab, $F(ab')_2$, single domain antibodies (DABs), Fv, scFv (single chain Fv), and the like. The techniques for preparing and using various antibody-based constructs and fragments are well known in the art. Means for preparing and characterizing antibodies are also well known in the art (see, *e.g.,* Harlow *et al.,* 1988;)

**a. Antibody Generation**

In certain embodiments, the present disclosure involves antibodies. For example, all or part of a monoclonal may be used in determining the amino acid at position 389. As detailed above, in addition to antibodies generated against full length proteins, antibodies also may be generated in response to smaller constructs comprising epitopic core regions, including wild-type and mutant epitopes. The techniques for preparing and using various antibody-based constructs and fragments are well known in the art. Means for preparing and characterizing antibodies are also well known in the art (see, *e.g.,* Harlow and Lane, 1988).

Monoclonal antibodies (mAbs) are recognized to have certain advantages, *e.g.*, reproducibility and large-scale production, and their use is generally preferred. The disclosure thus provides monoclonal antibodies of the human, murine, monkey, rat, hamster, rabbit and even chicken origin.

The methods for generating monoclonal antibodies (mAbs) generally begin along the same lines as those for preparing polyclonal antibodies. Briefly, a polyclonal antibody may be prepared by immunizing an animal with an immunogenic polypeptide composition in accordance with the present disclosure and collecting antisera from that immunized animal. Alternatively, in some embodiments of the present invention, serum is collected from persons who may have been exposed to a particular antigen. Exposure to a particular antigen may occur a work environment, such that those persons have been occupationally exposed to a particular antigen and have developed polyclonal antibodies to a peptide, polypeptide, or protein. In some embodiments of the disclosure polyclonal serum from occupationally exposed persons is used to identify antigenic regions in the gelonin toxin through the use of immunodetection methods.

A wide range of animal species can be used for the production of antisera. Typically the animal used for production of antisera is a rabbit, a mouse, a rat, a hamster, a guinea pig or a goat. Because of the relatively large blood volume of rabbits, a rabbit is a preferred choice for production of polyclonal antibodies.

As is well known in the art, a given composition may vary in its immunogenicity. It is often necessary therefore to boost the host immune system, as may be achieved by coupling a peptide or polypeptide immunogen to a carrier. Exemplary and preferred carriers are keyhole limpet hemocyanin (KLH) and bovine serum albumin (BSA). Other albumins such as ovalbumin, mouse serum albumin or rabbit serum albumin also can be used as carriers. Means for conjugating a polypeptide to a carrier protein are well known in the art and include glutaraldehyde, m-maleimidobenzoyl-N-hydroxysuccinimide ester, carbodiimide and bis-biazotized benzidine.

As also well known in the art, the immunogenicity of a particular immunogen composition can be enhanced by the use of non-specific stimulators of the immune response, known as adjuvants. Suitable molecule adjuvants include all acceptable immunostimulatory compounds, such as cytokines, toxins or synthetic compositions.

Adjuvants that may be used include IL-1, IL-2, IL-4, IL-7, IL-12, $\gamma$-interferon, GMCSP, BCG, aluminum hydroxide, MDP compounds, such as thur-MDP and nor-MDP, CGP (MTP-PE), lipid A, and monophosphoryl lipid A (MPL). RIBI, which contains three components extracted from bacteria, MPL, trehalose dimycolate (TDM) and cell wall skeleton (CWS) in a 2% squalene/Tween 80 emulsion also is contemplated. MHC antigens may even be used. Exemplary, often preferred adjuvants include complete Freund's adjuvant (a non-specific stimulator of the immune response containing killed *Mycobacterium tuberculosis),* incomplete Freund's adjuvants and aluminum hydroxide adjuvant.

In addition to adjuvants, it may be desirable to coadminister biologic response modifiers (BRM), which have been shown to upregulate T cell immunity or downregulate suppressor cell activity. Such BRMs include, but are not limited to, Cimetidine (CIM; 1200 mg/d) (Smith/Kline, PA); low-dose Cyclophosphamide (CYP; 300 $mg/m^2$) (Johnson/Mead, NJ), cytokines such as $\gamma$-interferon, IL-2, or IL-12 or genes encoding proteins involved in immune helper functions, such as B-7.

The amount of immunogen composition used in the production of polyclonal antibodies varies upon the nature of the immunogen as well as the animal used for immunization. A variety of routes can be used to administer the immunogen (subcutaneous, intramuscular, intradermal, intravenous and intraperitoneal). The production of polyclonal antibodies may be monitored by sampling blood of the immunized animal at various points following immunization.

A second, booster injection also may be given. The process of boosting and titering is repeated until a suitable titer is achieved. When a desired level of immunogenicity is obtained, the immunized animal can be bled and the serum isolated and stored, and/or the animal can be used to generate mAbs.

mAbs may be readily prepared through use of well-known techniques, such as those exemplified in U.S. Patent 4,196,265. Typically, this technique involves immunizing a suitable animal with a selected immunogen composition, *e.g.*, a purified or partially purified polypeptide, peptide or domain, be it a wild-type or mutant composition. The immunizing composition is administered in a manner effective to stimulate antibody producing cells.

mAbs may be further purified, if desired, using filtration, centrifugation and various chromatographic methods such as HPLC or affinity chromatography. Fragments of the monoclonal antibodies of the invention can be obtained from the monoclonal antibodies so produced by methods which include digestion with enzymes, such as pepsin or papain, and/or by cleavage of disulfide bonds by chemical reduction. Alternatively, monoclonal antibody fragments encompassed by the present invention can be synthesized using an automated peptide synthesizer.

It also is contemplated that a molecular cloning approach may be used to generate mAbs. For this, combinatorial immunoglobulin phagemid libraries are prepared from RNA isolated from the spleen of the immunized animal, and phagemids expressing appropriate antibodies are selected by panning using cells expressing the antigen and control cells. The advantages of this approach over conventional hybridoma techniques are that approximately $10^4$ times as many antibodies can be produced and screened in a single round, and that new specificities are generated by H and L chain combination which further increases the chance of finding appropriate antibodies.

**b. Immunodetection Methods**

As discussed, in some embodiments, the present disclosure concerns immunodetection methods for binding, purifying, removing, determining, and/or otherwise detecting biological components such as antigenic regions on polypeptides and peptides. The immunodetection methods of the present disclosure can be used to identify antigenic regions of a peptide, polypeptide, or protein that has therapeutic implications, particularly in reducing the immunogenicity or antigenicity of the peptide, polypeptide, or protein in a target subject.

Immunodetection methods include enzyme linked immunosorbent assay (ELISA), radioimmunoassay (RIA), immunoradiometric assay, fluoroimmunoassay, chemiluminescent assay, bioluminescent assay, and Western blot, though several others are well known to those of ordinary skill. The steps of various useful immunodetection methods have been described in the scientific literature, such as, *e.g.,* Doolittle *et al.,* 1999; Gulbis *et al.,* 1993; De Jager *et al.,* 1993; and Nakamura *et al.,* 1987.

In general, the immunobinding methods include obtaining a sample suspected of containing a protein, polypeptide and/or peptide, and contacting the sample with a first antibody, monoclonal or polyclonal, in accordance with the present invention, as the case may be, under conditions effective to allow the formation of immunocomplexes.

These methods include methods for purifying a protein, polypeptide and/or peptide from organelle, cell, tissue or organism's samples. In these instances, the antibody removes the antigenic protein, polypeptide and/or peptide component from a sample. The antibody will preferably be linked to a solid support, such as in the form of a column matrix, and the sample suspected of containing the protein, polypeptide and/or peptide antigenic component will be applied to the immobilized antibody. The unwanted components will be washed from the column, leaving the antigen immunocomplexed to the immobilized antibody to be eluted.

The immunobinding methods also include methods for detecting and quantifying the amount of an antigen component in a sample and the detection and quantification of any immune complexes formed during the binding process. Here, one would obtain a sample suspected of containing an antigen or antigenic domain, and contact the sample with an antibody against the antigen or antigenic domain, and then detect and quantify the amount of immune complexes formed under the specific conditions.

In terms of antigen detection, the biological sample analyzed may be any sample that is suspected of containing an antigen or antigenic domain, such as, for example, a tissue section or specimen, a homogenized tissue extract, a cell, an organelle, separated and/or purified forms of any of the above antigen-containing compositions, or even any biological fluid that comes into contact with the cell or tissue, including blood and/or serum..

Contacting the chosen biological sample with the antibody under effective conditions and for a period of time sufficient to allow the formation of immune complexes (primary immune complexes) is generally a matter of simply adding the antibody composition to the sample and incubating the mixture for a period of time long enough for the antibodies to form immune complexes with, *i.e.,* to bind to, any antigens present. After this time, the sample-antibody composition, such as a tissue section, ELISA plate, dot blot or western blot, will generally be washed to remove any non-specifically bound antibody species, allowing only those antibodies specifically bound within the primary immune complexes to be detected.

In general, the detection of immunocomplex formation is well known in the art and may be achieved through the application of numerous approaches. These methods are generally based upon the detection of a label or marker, such as any of those radioactive, fluorescent, biological and enzymatic tags. U.S. Patents concerning the use of such labels include 3,817,837; 3,850,752; 3,939,350; 3,996,345; 4,277,437; 4,275,149 and 4,366,241. Of course, one may find additional advantages through the use of a secondary binding ligand such as a second antibody and/or a biotin/avidin ligand binding arrangement, as is known in the art.

The antibody employed in the detection may itself be linked to a detectable label, wherein one would then simply detect this label, thereby allowing the amount of the primary immune complexes in the composition to be determined. Alternatively, the first antibody that becomes bound within the primary immune complexes may be detected by means of a second binding ligand that has binding affinity for the antibody. In these cases, the second binding ligand may be linked to a detectable label. The second binding ligand is itself often an antibody, which may thus be termed a "secondary" antibody. The primary immune complexes are contacted with the labeled, secondary binding ligand, or antibody, under effective conditions and for a period of time sufficient to allow the formation of secondary immune complexes. The secondary immune complexes are then generally washed to remove any non-specifically bound labeled secondary antibodies or ligands, and the remaining label in the secondary immune complexes is then detected.

Further methods include the detection of primary immune complexes by a two step approach. A second binding ligand, such as an antibody, that has binding affinity for the antibody is used to form secondary immune complexes, as described above. After washing, the secondary immune complexes are contacted with a third binding ligand or antibody that has binding affinity for the second antibody, again under effective conditions and for a period of time sufficient to allow the formation of immune complexes (tertiary immune complexes). The third ligand or antibody is linked to a detectable label, allowing detection of the tertiary immune complexes thus formed. This system may provide for signal amplification if this is desired.

One method of immunodetection designed by Charles Cantor uses two different antibodies. A first step biotinylated, monoclonal or polyclonal antibody is used to detect the target antigen(s), and a second step antibody is then used to detect the biotin attached to the complexed biotin. In that method the sample to be tested is first incubated in a solution containing the first step antibody. If the target antigen is present, some of the antibody binds to the antigen to form a biotinylated antibody/antigen complex. The antibody/antigen complex is then amplified by incubation in successive solutions of streptavidin (or avidin), biotinylated DNA, and/or complementary biotinylated DNA, with each step adding additional biotin sites to the antibody/antigen complex. The amplification steps are repeated until a suitable level of amplification is achieved, at which point the sample is incubated in a solution containing the second step antibody against biotin. This second step antibody is labeled, as for example with an enzyme that can be used to detect the presence of the antibody/antigen complex by histoenzymology using a chromogen substrate. With suitable amplification, a conjugate can be produced which is macroscopically visible.

Another known method of immunodetection takes advantage of the immuno-PCR (Polymerase Chain Reaction) methodology. The PCR method is similar to the Cantor method up to the incubation with biotinylated DNA, however, instead of using multiple rounds of streptavidin and biotinylated DNA incubation, the DNA/biotin/streptavidin/antibody complex is washed out with a low pH or high salt buffer that releases the antibody. The resulting wash solution is then used to carry out a PCR reaction with suitable primers with appropriate controls. At least in theory, the enormous amplification capability and specificity of PCR can be utilized to detect a single antigen molecule.

### i. ELISAs

As detailed above, immunoassays, in their most simple and/or direct sense, are binding assays. Certain preferred immunoassays are the various types of enzyme linked immunosorbent assays (ELISAs) and/or radioimmunoassays (RIA) known in the art. Immunohistochemical detection using tissue sections is also particularly useful. However, it will be readily appreciated that detection is not limited to such techniques, and/or western blotting, dot blotting, FACS analyses, and/or the like may also be used.

In one exemplary ELISA, antibodies are immobilized onto a selected surface exhibiting protein affinity, such as a well in a polystyrene microtiter plate. Then, a test composition suspected of containing the antigen, such as a clinical sample, is added to the wells. After binding and/or washing to remove non-specifically bound immune complexes, the bound antigen may be detected. Detection is generally achieved by the addition of another antibody that is linked to a detectable label. This type of ELISA is a simple "sandwich ELISA." Detection may also be achieved by the addition of a second antibody, followed by the addition of a third antibody that has binding affinity for the second antibody, with the third antibody being linked to a detectable label. The ELISA may be based on differential binding of an antibody to a protein with Arg389 versus Gly389.

In another exemplary ELISA, the samples suspected of containing the antigen are immobilized onto the well surface and/or then contacted with antibodies. After binding and/or washing to remove non-specifically bound immune complexes, the bound anti-antibodies are detected. Where the initial antibodies are linked to a detectable label, the immune complexes may be detected directly. Again, the immune complexes may be detected using a second antibody that has binding affinity for the first antibody, with the second antibody being linked to a detectable label.

Another ELISA in which the antigens are immobilized, involves the use of antibody competition in the detection. In this ELISA, labeled antibodies against an antigen are added to the wells, allowed to bind, and/or detected by means of their label. The amount of an antigen in an unknown sample is then determined by mixing the sample with the labeled antibodies against the antigen during incubation with coated wells. The presence of an antigen in the sample acts to reduce the amount of antibody against the antigen available for binding to the well and thus reduces the ultimate signal. This is also appropriate for detecting antibodies against an antigen in an unknown sample, where the unlabeled antibodies bind to the antigen-coated wells and also reduces the amount of antigen available to bind the labeled antibodies.

Irrespective of the format employed, ELISAs have certain features in common, such as coating, incubating and binding, washing to remove non-specifically bound species, and detecting the bound immune complexes. These are described below.

In coating a plate with either antigen or antibody, one will generally incubate the wells of the plate with a solution of the antigen or antibody, either overnight or for a specified period of hours. The wells of the plate will then be washed to remove incompletely adsorbed material. Any remaining available surfaces of the wells are then "coated" with a nonspecific protein that is antigenically neutral with regard to the test antisera. These include bovine serum albumin (BSA), casein or solutions of milk powder. The coating allows for blocking of nonspecific adsorption sites on the immobilizing surface and thus reduces the background caused by nonspecific binding of antisera onto the surface.

In ELISAs, it is probably more customary to use a secondary or tertiary detection means rather than a direct procedure. Thus, after binding of a protein or antibody to the well, coating with a non-reactive material to reduce background, and washing to remove unbound material, the immobilizing surface is contacted with the biological sample to be tested under conditions effective to allow immune complex (antigen/antibody) formation. Detection of the immune complex then requires a labeled secondary binding ligand or antibody, and a secondary binding ligand or antibody in conjunction with a labeled tertiary antibody or a third binding ligand.

"Under conditions effective to allow immune complex (antigen/antibody) formation" means that the conditions preferably include diluting the antigens and/or antibodies with solutions such as BSA, bovine gamma globulin (BGG) or phosphate buffered saline (PBS)/Tween. These added agents also tend to assist in the reduction of nonspecific background.

The "suitable" conditions also mean that the incubation is at a temperature or for a period of time sufficient to allow effective binding. Incubation steps are typically from about 1 to 2 to 4 hours or so, at temperatures preferably on the order of 25°C to 27°C, or may be overnight at about 4°C or so.

Following all incubation steps in an ELISA, the contacted surface is washed so as to remove non-complexed material. An example of a washing procedure includes washing with a solution such as PBS/Tween, or borate buffer. Following the formation of specific immune complexes between the test sample and the originally bound material, and subsequent washing, the occurrence of even minute amounts of immune complexes may be determined.

To provide a detecting means, the second or third antibody will have an associated label to allow detection. This may be an enzyme that will generate color development upon incubating with an appropriate chromogenic substrate. Thus, for example, one will desire to contact or incubate the first and second immune complex with a urease, glucose oxidase, alkaline phosphatase or hydrogen peroxidase-conjugated antibody for a period of time and under conditions that favor the development of further immune complex formation (*e.g.*, incubation for 2 hours at room temperature in a PBS-containing solution such as PBS-Tween).

After incubation with the labeled antibody, and subsequent to washing to remove unbound material, the amount of label is quantified, *e.g.*, by incubation with a chromogenic substrate such as urea, or bromocresol purple, or 2,2'-azino-di-(3-ethyl-benzthiazoline-6-sulfonic acid (ABTS), or $H_2O_2$, in the case of peroxidase as the enzyme label. Quantification is then achieved by measuring the degree of color generated, *e.g.*, using a visible spectra spectrophotometer.

### ii. Immunohistochemistry

The antibodies of the present disclosure may also be used in conjunction with both fresh-frozen and/or formalin-fixed, paraffin-embedded tissue blocks prepared for study by immunohistochemistry (IHC). For example, immunohistochemistry may be utilized to characterize Fortilin or to evaluate the amount Fortilin in a cell. The method of preparing tissue blocks from these particulate specimens has been successfully used in previous IHC studies of various prognostic factors, and/or is well known to those of skill in the art (Brown *et al.,* 1990; Abbondanzo *et al.,* 1990; Allred *et al.,* 1990).

Briefly, frozen-sections may be prepared by rehydrating 50 mg of frozen "pulverized" tissue at room temperature in phosphate buffered saline (PBS) in small plastic capsules; pelleting the particles by centrifugation; resuspending them in a viscous embedding medium (OCT); inverting the capsule and/or pelleting again by centrifugation; snap-freezing in -70°C isopentane; cutting the plastic capsule and/or removing the frozen cylinder of tissue; securing the tissue cylinder on a cryostat microtome chuck; and/or cutting 25-50 serial sections.

Permanent-sections may be prepared by a similar method involving rehydration of the 50 mg sample in a plastic microfuge tube; pelleting; resuspending in 10% formalin for 4 hours fixation; washing/pelleting; resuspending in warm 2.5% agar; pelleting; cooling in ice water to harden the agar; removing the tissue/agar block from the tube; infiltrating and/or embedding the block in paraffin; and/or cutting up to 50 serial permanent sections.

## V. Examples

The following examples are included to further illustrate various aspects of the invention. It should be appreciated by those of skill in the art that the techniques disclosed in the examples which follow represent techniques and/or compositions discovered by the inventor to function well in the practice of the invention, and thus can be considered to constitute preferred modes for its practice.

**Example 1: Materials and Methods**

**Sources of Canine DNA Samples.** Individual DNA samples from normal and DM-affected dogs were obtained from the Canine Health Information Center (CHIC), DNA Repository (the world wide web at caninehealthinfo.org/), and from DNA collections at the University of Missouri, the Broad Institute of Harvard and Massachusetts Institute of Technology, and the University of Pennsylvania. The other-breeds control group consisted of 2 randomly selected and unrelated individuals from 60 dog breeds, in which DM is rarely, if ever, reported.

**Diagnosis of DM.** The DM cases involved privately owned dogs that were referred to one of the participating Colleges of Veterinary Medicine. Depending on the availability of tissues and clinical information, diagnoses of DM were made on the basis of 4 sets of criteria of varying stringency. The inventors considered confirmation of DM by histopathology to be the most stringent criterion for DM diagnosis (stringency level 1); however, spinal cords were not available from all dogs in the study. The diagnoses of DM at stringency levels 2 and 3 were based on the presence of typical clinical signs and the absence of a compressive lesion detectable by MRI (level 2) or myelography (level 3). The least stringent diagnoses (level 4) were based solely on suggestive clinical signs, which included progressive upper motor neuron paresis and general proprioceptive ataxia.

**Association Mapping.** GWA analysis was undertaken by using the Affymetrix Canine Genome 2.0 Array "Platinum Panel" containing 49,663 SNP markers in 38 cases (diagnostic classification: score 1 $n$ = 21, 2 $n$ = 5, 3 $n$ = 2, 4 $n$ = 10) and 17 controls. SNP genotypes were obtained following the human 500K array protocol, but with a smaller hybridization volume to allow for the smaller surface area of the canine array as described elsewhere (Karlsson *et al.,* 2007). Detailed information on the arrays is available at the world-wide-web at broad.mit.edu/node/456. Case-control GWA mapping was evaluated by using PLINK (Purcell *et al.,* 2007), followed by the identification of a region of homozygosity in affected individuals based on SNP genotypes. Fine mapping was performed by using MassARRAY (Sequenom) assays for 63 SNPs in 207 samples from 5 breeds as previously described (Karlsson *et al.,* 2007). Haplotype analysis was performed with Haploview (Barrett *et al.,* 2005). The sources of samples used for fine mapping are identified in FIG. 1C.

**Resequencing and Genotyping.** Exons 2 to 5 of canine *SOD1* were resequenced after PCR amplification of genomic DNA from DM-affected and normal dogs. Oligonucleotide primers were designed from sequences flanking these exons from build 2.1 of the canine genome reference sequence (world-wide-web at ncbi.nlm.nih.gov/projects/mapview/map_search.cgi?taxid_9615). Because exon 1 of *SOD1* is not represented in build 2.1, the inventors used RT-PCR to amplify exon 1-containing RNA segments in total RNA extracted from blood from DM-affected and normal dogs with thePAXgeneBloodRNAKit (Qiagen). The RT-PCR primers were designed from the consensus sequence produced from an alignment of all canine exon 1-containing expressed sequence tags in GenBank. Purified PCR and RT-PCR amplicons were sequenced with an Applied Biosystems 3730x1 DNA analyzer. Some of the canine DNA samples were genotyped at the SOD1:c.118G>A locus by pyrosequencing with a PSQ 96 Pyrosequencer.

The PCR primers were 5'-biotinyl-AGTGGGCCTGTTGTGGTATCA with CTCCAAACTGATGGACGTGGAAT, and AATCCATGCTCGCCTT was used for the sequencing primer.Genotype distributions for affected and control samples were compared by using 2 x 2 contingency tables, in which A/G and G/G genotypes were pooled under the assumption of autosomal recessive inheritance. A TaqMan® allelic discrimination assay was used to genotype other canine DNA samples. The PCR primer sequences for this assay were GTGGGCCTGTTGTGGTATCA with CAAACTGATGGACGTGGAATCC and the competing fluorescent labeled probe sequences were VIC- CTCGCCTTTAGTCAGC (mutant) and FAM- CGCCTTCAGTCAGC. Fisher's exact 1-tailed test was used to test for the independence of the genotype classes between the case and control samples.

**Histopathology, Immunohistopathology, and Electrodiagnostic Testing.** Standard procedures were used for histopathology, immunohistopathology, and electrodiagnostic testing. Samples used for SOD1 immunohistochemistry were coded, and micrographs of spinal cord motor neurons were obtained in a masked manner. A second masked evaluator classified the neurons in the micrographs according to the presence and appearance of SOD1-positive inclusions based on the following categories: well-defined dark staining clumps, well-defined light staining clumps, poorly defined light staining regions, and no staining or diffuse light staining similar to the background staining; 6 to 9 sections from each cord were examined.

**Example 2: Results**

**Mapping the DM Locus and Identification of a SOD1 Missense Mutation.** Genome-wide association (GWA) mapping of DM was performed with 38 cases and 17 controls older than 6 years of age (mean age = 9.4 years) from the Pembroke Welsh corgi breed by using the Affymetrix Canine Genome 2.0 Array. The strongest association was detected on CFA31 ($p_{raw} = 1 \times 10^{-5}$; $p_{genome} = 0.18$), with weaker signals on 4 other chromosomes, suggesting modifiers or population substructure (FIG. 1A).Within the associated CFA31 region, all affected dogs were homozygous for a common haplotype from 28.91 to 29.67 Mb (CanFam2.0), which contains 3 genes: *SOD1, TIAM1,* and *SFRS15*. Clinical similarities between DM and ALS made *SOD1* a viable candidate gene. Resequencing *SOD1* from normal and DM-affected dogs revealed a G to A transition in exon 2 that predicts an E40K missense mutation. The 55 corgi DNA samples were genotyped for the SOD1:c.118G>A polymorphism. All 38 samples from affected corgis were homozygous for the A allele, whereas the 17-sample asymptomatic control group consisted of 10 A/A homozygotes, 6 A/G heterozygotes and 1 G/G homozygote.

To verify the localization of the DM mutation, the inventors fine mapped 90 SNPs across a 1.9-Mb region from 29.04 to 30.97 Mb in 5 breeds, which segregate for DM (FIG. 1B). Affected dogs from all 5 breeds share a 5-SNP haplotype (maximum 195 kB in size), which contains the E40K mutation (FIG. 1C). This haplotype is also present in dogs that do not have the mutation. No other SNP or haplotype in the region is both shared across all breeds and concordant with recessive inheritance. Thus, the significant proportion of A/A mutant homozygotes among the controls and the presence of E40K mutation on an ancestral haplotype still present in the population may explain the relatively weak GWA to this region. Nonetheless, the presented genetic data strongly links the E40K mutation with the disease.

**Additional Genotyping Confirms an Association Between DM and Homozygosity for the SOD1 Missense Mutation.** The Pembroke Welsh corgis used for GWA, plus an additional 64 Pembroke Welsh corgis and 418 representatives from 4 other breeds, were genotyped for the SOD1:c.118G>A polymorphism. Across all breeds, 100 of the samples were from dogs diagnosed with DM; however, these diagnoses were not all based on equally stringent criteria (see Methods). Table 1 shows the distribution of genotypes for all 537 representatives of the 5 breeds by diagnostic class. Significant associations between the DM phenotype and homozygosity for the A allele were detected when all 5 affected breeds were jointly analyzed ($P$ = 2.93E-19) and when each breed was analyzed individually (Table 1). The frequency of the A allele in a separate "other-breeds" control group, consisting of samples from dog breeds in whichDMis rarely diagnosed, was significantly lower than in the controls from the affected breeds (Table 1). The 4 dogs in the study that were classified as affected, but were not A/A homozygotes, were all diagnosed by using the least stringent criteria and may be phenocopies.

**Table 1 - Associations Between DM Phenotype and Homozygosity**

| **Breed** | **DM Stringency 1** | **DM Stringencies 1&2** | **DM Stringencies 1-3** | **DM Stringencies 1-4** | **Affected Breed Controls** | **Other Breed Controls** |
|---|---|---|---|---|---|---|
| | AA/AG/GG | AA/AG/GG | AA/AG/GG | AA/AG/GG | AA/AG/GG | AA/AG/GG |
| Boxer | 9/0/0* | 10/0/0* | 12/0/0* | 22/0/0** | 86/57/14 | - |
| Chesapeake Bay Retr. | 7/0/0** | 9/0/0** | 9/0/0** | 10/0/0** | 7/25/21 | - |
| German Shepherd | 2/0/0* | 4/0/0** | 4/0/0** | 4/0/1** | 7/30/84 | - |
| Pembroke Welsh Corgi | 25/0/0** | 30/0/0** | 35/0/0** | 50/1/1** | 44/14/9 | - |
| Rhodesian Ridgeback | 3/0/0* | 6/0/0** | 6/0/0** | 10/1/0** | 4/15/21 | - |
| All Affected Dogs | 46/0/0*** | 59/0/0*** | 66/0/0*** | 96/2/2*** | 148/141/148 | 0/5/115*** |

* - Different than breed-specific controls at P < 0.01
** - Different than breed-specific controls at P < 0.001
*** - Different than all affected breed controls at P < 0.00001
DM Stringency 1 = Histopathologically confirmed
DM Stringency 2 = MRI presumptive diagnosis
DM Stringency 3 = Myelography presumptive diagnosis
DM Stringency 4 = suggestive clinical signs for a presumptive diagnosis

**Dogs with DM Exhibit Symptoms and Histopathologic and Immunohistopathologic Lesions Similar to Those in ALS Patients.** The diagnosis of DM was confirmed by histopathologic examination of spinal cord sections in 46 dogs. Affected dogs had lesions in the posterior and lateral columns (FIGS. 2A-B). Surviving spinal cord neurons from 7 DM-affected dogs and 10 similarly aged asymptomatic control dogs were examined by immunohistopathology. All 7 of the DM-affected dogs were A/A homozygotes, and all contained cytoplasmic inclusions, which, when stained with anti-SOD1 antibodies, appeared as well-defined dark clumps. In contrast, no staining or diffuse light staining similar to thebackground staining was found in cells in the spinal cords from all 5 of the control dogs with the G/G genotype and in 3 of the 5 A/G heterozygous controls. Intermediate levels of cytoplasmic staining with anti-SOD1 antibodies were observed in the spinal cords from the remaining 2 heterozygous control dogs (FIGS. 3A-I).

Although most dogs with DM are euthanized at an early stage of the disease when upper motor neuron pathology predominates, the owners of some dogs in the study elected to maintain their dogs until the disease was more advanced. Dogs with advanced DM exhibited clinical signs of lower motor neuron disease, including ascending flaccid tetraparesis, generalized muscle atrophy, and hyporeflexia in all limbs. One DM affected Pembroke Welsh corgi was euthanized 48 months after the onset of clinical signs due to swallowing difficulties, which suggests that the disease can progress to bulbar signs. In the early disease stage, no spontaneous activity was detected by electromyography (EMG), and nerve conduction velocities were within normal limits. In the late disease stage, EMG revealed multifocal spontaneous activity in the distal appendicular musculature. Fibrillation potentials and sharp waves were the most common waveforms recorded. Compared with canine-specific reference ranges (Walker *et al.*, 1979), compound muscle action potentials (M waves) recorded in the tibial and ulnar nerves showed temporal dispersion and decreases in amplitudes, and motor nerve conduction velocities were decreased (data not shown). Muscle specimens from dogs with advanced DM showed excessive variability in myofiber size with large and small groups of atrophic fibers typical of denervation (FIG. 4A). Peripheral nerve specimens from these dogs showed nerve fiber loss as indicated by axonal degeneration, endoneurial fibrosis, numerous inappropriately thinly myelinated fibers, and secondary demyelination (FIG. 4C).

**Age-Related Incomplete Penetrance.** Many of the 148 A/A homozygotes in the "affected-breeds" control group were younger when sampled than the typical age at onset of clinical signs ofDM (FIG. 5). Some of these dogs may develop DM when they grow older. Nonetheless, the considerable number of A/A homozygotes among the older affected-breed controls that exhibited noclinical signs of DM (FIG. 5) indicates that the penetrance among A/A homozygotes is incomplete, possibly due to modifier loci, environmental factors, and/or because the A/A homozygotes die from other causes before the clinical signs become apparent.

To determine if the *SOD1* A allele occurs in other breeds in addition to the 5 breeds that werde the subject of our early experiments, we genotyped over 6600 canine DNA samples selected from a collection of over 60,000 DNA samples held at the University of Missouri (Table 2). The A allele was detected in 57 of 147 breeds. Among the breeds in which the A allele was detected, allele frequencies varied from 90% in wire fox terriers to 1% in Labrador retrievers.

**Table 2 - Frequencies of CDM-associated SOD1 allele in various dog breeds**

| Breed | TOTAL | Normal | Carrier | At Risk | Allele Frequency (%) |
|---|---|---|---|---|---|
| Fox Terrier - Wire | 39 | 1 | 6 | 32 | 90 |
| Pembroke Welsh Corgi | 626 | 28 | 173 | 425 | 81 |
| Boxer | 711 | 84 | 240 | 387 | 71 |
| hybrid/mix-breed | 44 | 18 | 5 | 21 | 53 |
| Cavalier King Charles Spaniel | 11 | 1 | 9 | 1 | 50 |
| Dutch Shepherd | 1 | 0 | 1 | 0 | 50 |
| American Water Spaniel | 47 | 15 | 21 | 11 | 46 |
| Chesapeake Bay Retriever | 553 | 188 | 235 | 130 | 45 |
| Bernese Mountain Dog | 38 | 14 | 17 | 7 | 41 |
| American Eskimo Dog | 10 | 3 | 6 | 1 | 40 |
| Pug | 13 | 6 | 4 | 3 | 38 |
| Kerry Blue Terrier | 153 | 70 | 54 | 29 | 37 |
| Canaan Dog | 113 | 51 | 50 | 12 | 33 |
| Rhodesian Ridgeback | 514 | 237 | 218 | 59 | 33 |
| Cardigan Welsh Corgi | 79 | 36 | 35 | 8 | 32 |
| German Shepherd Dog | 445 | 247 | 112 | 86 | 32 |
| Tibetan Terrier | 43 | 23 | 15 | 5 | 29 |
| Bloodhound | 32 | 16 | 14 | 2 | 28 |
| Welsh Terrier | 52 | 29 | 18 | 5 | 27 |
| French Bulldog | 33 | 21 | 9 | 3 | 23 |
| Kuvasz | 60 | 39 | 16 | 5 | 22 |
| Chow Chow | 22 | 14 | 7 | 1 | 20 |
| Chinese Crested | 30 | 19 | 11 | 0 | 18 |
| Irish Setter | 31 | 21 | 9 | 1 | 18 |

(continued)

| Breed | TOTAL | Normal | Carrier | At Risk | Allele Frequency (%) |
|---|---|---|---|---|---|
| Australian Terrier | 3 | 2 | 1 | 0 | 17 |
| Airedale Terrier | 30 | 21 | 9 | 0 | 15 |
| Dalmatian | 33 | 32 | 1 | 0 | 15 |
| Sealyham Terrier | 30 | 21 | 9 | 0 | 15 |
| Pomeranian | 29 | 22 | 6 | 1 | 14 |
| Jack Russell Terrier | 27 | 21 | 5 | 1 | 13 |
| Mastiff (English Mastiff) | 31 | 24 | 6 | 1 | 13 |
| Nova Scotia Duck Tolling Retriever | 32 | 25 | 6 | 1 | 13 |
| Australian Shepherd | 34 | 27 | 6 | 1 | 12 |
| Belgian Sheepdog | 4 | 3 | 1 | 0 | 12 |
| Collie | 29 | 22 | 7 | 0 | 12 |
| English Springer Spaniel | 72 | 56 | 14 | 2 | 12 |
| Great Pyrenees | 26 | 21 | 4 | 1 | 12 |
| Border Collie | 28 | 25 | 1 | 2 | 9 |
| Shetland Sheepdog | 29 | 24 | 5 | 0 | 9 |
| Shih Tzu | 8 | 3 | 4 | 1 | 9 |
| Soft Coated Wheaten Terrier | 29 | 24 | 5 | 0 | 9 |
| Coton de Tulear | 30 | 26 | 4 | 0 | 7 |
| Harrier | 28 | 24 | 4 | 0 | 7 |
| English Shepherd | 27 | 24 | 3 | 0 | 6 |
| Finnish Lapphund | 31 | 27 | 4 | 0 | 6 |
| Poodle-Standard | 77 | 70 | 5 | 2 | 6 |
| Staffordshire Bull Terrier | 33 | 31 | 0 | 2 | 6 |
| Australian Cattle Dog | 46 | 41 | 5 | 0 | 5 |
| Beagle | 31 | 28 | 3 | 0 | 5 |
| Clumber Spaniel | 33 | 30 | 3 | 0 | 5 |
| Irish Terrier | 30 | 27 | 3 | 0 | 5 |
| German Pinscher | 30 | 28 | 2 | 0 | 3 |
| Irish Wolfhound | 31 | 29 | 2 | 0 | 3 |
| Finnish Spitz | 32 | 31 | 1 | 0 | 2 |
| Keeshond | 29 | 28 | 1 | 0 | 2 |
| Rottweiler | 26 | 25 | 1 | 0 | 2 |
| Labrador Retriever | 59 | 58 | 1 | 0 | 1 |
| Afghan Hound | 1 | 1 | 0 | 0 | 0 |
| Akita | 33 | 33 | 0 | 0 | 0 |
| Alaskan Husky | 1 | 1 | 0 | 0 | 0 |
| Alaskan Malamute | 36 | 36 | 0 | 0 | 0 |
| American Bulldog | 31 | 31 | 0 | 0 | 0 |
| American Foxhound | 1 | 1 | 0 | 0 | 0 |
| American Staffordshire Terrier | 6 | 6 | 0 | 0 | 0 |
| Basenji | 33 | 33 | 0 | 0 | 0 |
| Basset Hound | 28 | 28 | 0 | 0 | 0 |
| Bearded Collie | 29 | 29 | 0 | 0 | 0 |
| Beauceron | 2 | 2 | 0 | 0 | 0 |
| Bedlington Terrier | 29 | 29 | 0 | 0 | 0 |
| Belgian Malinois | 4 | 4 | 0 | 0 | 0 |
| Bichon Frise | 9 | 9 | 0 | 0 | 0 |
| Border Terrier | 30 | 30 | 0 | 0 | 0 |
| Borzoi | 2 | 2 | 0 | 0 | 0 |
| Boston Terrier | 6 | 6 | 0 | 0 | 0 |

(continued)

| Breed | TOTAL | Normal | Carrier | At Risk | Allele Frequency (%) |
|---|---|---|---|---|---|
| Bouvier des Flandres | 45 | 45 | 0 | 0 | 0 |
| Briard | 6 | 6 | 0 | 0 | 0 |
| Brittany | 31 | 31 | 0 | 0 | 0 |
| Bull Terrier | 1 | 1 | 0 | 0 | 0 |
| Cairn Terrier | 15 | 15 | 0 | 0 | 0 |
| Chinese Shar Pei | 26 | 26 | 0 | 0 | 0 |
| Chinook | 26 | 26 | 0 | 0 | 0 |
| Cocker Spaniel (American) | 28 | 28 | 0 | 0 | 0 |
| Curly Coated Retriever | 30 | 30 | 0 | 0 | 0 |
| Dachshund | 44 | 44 | 0 | 0 | 0 |
| Dandie Dinmont Terrier | 31 | 31 | 0 | 0 | 0 |
| Doberman Pinscher | 31 | 31 | 0 | 0 | 0 |
| Dogue du Bordeaux | 6 | 6 | 0 | 0 | 0 |
| English Cocker Spaniel | 28 | 28 | 0 | 0 | 0 |
| English Foxhound | 1 | 1 | 0 | 0 | 0 |
| English Setter | 27 | 27 | 0 | 0 | 0 |
| Field Spaniel | 30 | 30 | 0 | 0 | 0 |
| Flat-Coated Retriever | 30 | 30 | 0 | 0 | 0 |
| Fox Terrier - Smooth | 12 | 12 | 0 | 0 | 0 |
| German Shorthaired Pointer | 29 | 29 | 0 | 0 | 0 |
| Giant Schnauzer | 32 | 32 | 0 | 0 | 0 |
| Golden Retriever | 74 | 72 | 0 | 2 | 3 |
| Gordon Setter | 31 | 31 | 0 | 0 | 0 |
| Great Dane | 27 | 27 | 0 | 0 | 0 |
| Greater Swiss Mountain Dog | 36 | 36 | 0 | 0 | 0 |
| Havanese | 11 | 11 | 0 | 0 | 0 |
| Ibizan Hound | 31 | 31 | 0 | 0 | 0 |
| Icelandic Sheepdog | 30 | 30 | 0 | 0 | 0 |
| Irish Red & White Setter | 13 | 13 | 0 | 0 | 0 |
| Irish Water Spaniel | 28 | 28 | 0 | 0 | 0 |
| Italian Greyhound | 31 | 31 | 0 | 0 | 0 |
| Japanese Chin | 2 | 2 | 0 | 0 | 0 |
| Leonberger | 31 | 31 | 0 | 0 | 0 |
| Lhasa Apso | 17 | 17 | 0 | 0 | 0 |
| Lowchen | 33 | 33 | 0 | 0 | 0 |
| Manchester Terrier (Toy & Std) | 30 | 30 | 0 | 0 | 0 |
| Miniature Bull Terrier | 32 | 32 | 0 | 0 | 0 |
| Miniature Pinscher | 0 | 0 | 0 | 0 | 0 |
| Miniature Schnauzer | 3 | 3 | 0 | 0 | 0 |
| Neapolitan Mastiff | 12 | 12 | 0 | 0 | 0 |
| Newfoundland | 25 | 25 | 0 | 0 | 0 |
| Norwegian Lundehund | 1 | 1 | 0 | 0 | 0 |
| Norwich Terrier | 2 | 2 | 0 | 0 | 0 |
| Old English Sheepdog | 34 | 34 | 0 | 0 | 0 |
| Otterhound | 29 | 29 | 0 | 0 | 0 |
| Papillon | 1 | 1 | 0 | 0 | 0 |
| Petit Basset Griffon Vendeen | 32 | 32 | 0 | 0 | 0 |
| Parson Russell Terrier | 2 | 2 | 0 | 0 | 0 |
| Pharaoh Hound | 30 | 30 | 0 | 0 | 0 |
| Pointer | 28 | 28 | 0 | 0 | 0 |

(continued)

| Breed | TOTAL | Normal | Carrier | At Risk | Allele Frequency (%) |
|---|---|---|---|---|---|
| Portuguese Water Dog | 33 | 33 | 0 | 0 | 0 |
| Pyrenean Shepherd | 29 | 29 | 0 | 0 | 0 |
| Saint Bernard | 27 | 27 | 0 | 0 | 0 |
| Saluki | 2 | 2 | 0 | 0 | 0 |
| Samoyed | 30 | 30 | 0 | 0 | 0 |
| Schipperke | 28 | 28 | 0 | 0 | 0 |
| Scottish Deerhound | 36 | 36 | 0 | 0 | 0 |
| Scottish Terrier | 27 | 27 | 0 | 0 | 0 |
| Shiba Inu | 8 | 8 | 0 | 0 | 0 |
| Siberian Husky | 54 | 54 | 0 | 0 | 0 |
| Spinone Italiano | 2 | 2 | 0 | 0 | 0 |
| Standard Schnauzer | 25 | 25 | 0 | 0 | 0 |
| Sussex Spaniel | 28 | 28 | 0 | 0 | 0 |
| Swedish Valhund | 3 | 3 | 0 | 0 | 0 |
| Tibetan Mastiff | 10 | 10 | 0 | 0 | 0 |
| Tibetan Spaniel | 2 | 2 | 0 | 0 | 0 |
| Toy Fox Terrier | 2 | 2 | 0 | 0 | 0 |
| Vizsla | 28 | 28 | 0 | 0 | 0 |
| Weimeraner | 29 | 29 | 0 | 0 | 0 |
| Welsh Springer Spaniel | 32 | 32 | 0 | 0 | 0 |
| West Highland White Terrier | 3 | 3 | 0 | 0 | 0 |
| Whippet | 39 | 39 | 0 | 0 | 0 |
| Wirehaired Pointing Griffon | 5 | 5 | 0 | 0 | 0 |
| **Totals** | **6631** | **3989** | **1412** | **1230** | |

Example 3: Discussion

So far the DM-associated A allele has been detected in 57 different dog breeds. The wide spread occurance of the A allele in many unrelated dog breeds indicates that the A allele pre-dates the development of the various dog breeds. This indicates that the A allele is likely to occur to some extent in most or all dog breeds. Thus *SOD1* genotyping tests are applicable to all dog breeds and to mixed breed dogs.

SOD1 functions as a homodimer which converts superoxide radicals to hydrogen peroxide and molecular oxygen. Active sites in each subunit contain one copper ion and one zinc ion within an eight-stranded anti-parallel β-barrel (Tainer *et al.,* 1982). Amino acid position 40 lies within a connecting loop between β-strands 3 and 4 (Green *et al.,* 2002) which serves as the short Greek key connection between the two sets of β-strands that comprise the β-barrel (strands 1, 2, 3 and 6 and stands 4, 5, 7 and 8) (Boissinot *et al.,* 1997). Amino acid position 40 of human *SOD1* lies within a region that contains a cluster of missense mutations that are associated with human ALS (Deng *et al.,* 1993; Valentine *et al.,* 2005; Sandelin *et al.,* 2007), including E40G at a position orthologous to the canine E40K mutation (Sandelin *et al.,* 2007). Adjacent G41S and G41D mutations are believed to cause ALS by destabilizing both the SOD1 monomer and the dimerization interface (Lindberg *et al.,* 2005). The human E40G mutation and many other ALS-associated *SOD1* mutations reduce the net negative charge of the predicted protein product (Sandelin *et al.,* 2007; Shaw *et al.,* 2007). These SOD1 isoforms may be prone to the formation of toxic SOD1 aggregates because of reduced repulsive Coulombic forces, or because of increased interaction with anionic membrane surfaces (Sandelin *et al.,* 2007; Shaw *et al.,* 2007). Thus, the reduced net negative charge for canine SOD1 caused by the E40K mutation may be significant. A glutamate at a position corresponding to amino acid position 40 in canine SOD1 is conserved in 19 of 20 mammals identified in a Blastp query of the non-redundant protein sequences in the NCBI database (Table 3). The exception is equine SOD1 which, like the mutant canine allele, has a lysine at amino acid position 40. The inventors resequenced *SOD1* exon 2 from five unrelated horses and confirmed that horses commonly have a lysine at this position (data not shown). This unusual lysine may be tolerated because of ameliorating amino acid substitutions elsewhere in equine SOD1.

# TABLE 3

| Organism | Sequence | SEQ ID NO: |
|---|---|---|
| Dog (wild-type allele) | FVQKGSG-PVVVSGTITGLTEGEHGFHVHQFGDNTQGCTSAG | 10 |
| Rat | FEQKASGEPVVVSGQITGLTEGEHGFHVHQYGDNTQGCTTAG | 11 |
| Guinea pig | FEQKASGEPVVVSGQITGLTEGEHGFHVHQYGDNTQGCTTAG | 12 |
| Mouse | FEQKASGEPVVLSGQITGLTEGQHGFHVHQYGDNTQGCTSAG | 13 |
| Rabbit | FEQKGTG-PVVVKGRITGLTEGLHEFHVHQFGDNRQGCTSAG | 14 |
| Rhesus monkey | FEQKESNGPVKVWGSITGLTEGLHGFHVHQFGDNTQGCTSAG | 15 |
| Common gibbon | FEQKESNGPVKVYGRITGLTEGLHGFHVHQFGDNTQGCTSAG | 16 |
| Red deer | IRFEAKGNTVVVTGSITGLTEGDHGFHVHQFGDNTQGCTSAG | 17 |
| Cattle | IHFEAKGNTVVVTGSITGLTEGDHGFHVHQFGDNTQGCTSAG | 18 |
| Sheep | IRFEAKGDKVVVTGSITGLTEGDHGFHVHQFGDNTQGCTSAG | 19 |
| Crab-eating macaque | FEQKESNGPVKVWGSITGLTEGLHGYHVHQFGDNTQGCTSAG | 20 |
| Domestic yak | IHFEAKGDTVVVTGSITGLTEGDHGFHVHQFGDNTQGCTSAG | 21 |
| Goat | IHFEAKGDKVVVTGSITGLTEGDHGFHVHQFGDNTQGCTSAG | 22 |
| Pig | YFELKGEKTVLVTGTIKGLAEGDHGFHVHQFGDNTQGCTSAG | 23 |
| White-tufted-ear marmoset | FEQKESNGPVKVWGSITGLAEGLHGFHVHQFGDNTQGCTSAG | 24 |
| Brown capuchin | FEQKESNGPVKVWGSITGLAEGLHGFHVHQFGDNTQGCTSAG | 25 |
| Gray short-tailed opossum | FEQKQVGEPVELSGSIKGLAEGDHGFHVHEFGDNTQGCTSAG | 26 |
| Man | FEQKESNGPVKVWGSIKGLTEGLHGFHVHEFGDNTAGCTSAG | 27 |
| Orangutan | FEQKERNGPVKVWGSIEGLTEGLHGFHVHEFGDNTVGCTSAG | 28 |
| Horse | FEQQQEGGPVVLKGFIEGLTKGDHGFHVHEFGDNTQGCTTAG | 29 |
| Dog (mutant allele) | FVQKGSG-PVVVSGTITGLTKGEHGFHVHQFGDNTQGCTSAG | 30 |

The thoracic spinal cord in all DM affected dogs had bilateral regions of white matter sclerosis most prominent in the dorsal portion of the lateral funiculus lateral to the dorsal root entry zone. Few axons remained in the affected white matter. The sclerotic areas more darkly stained for GFAP than the surrounding white matter. There was a mild increase in the number of microglia in the affected tissue. Longitudinal sections of nerves had reduced axonal diameter and many axon sheaths appeared collapsed. Examined muscle specimens consisted mostly of fat and remaining muscle fibers were atrophic and somewhat angular. No inclusions were observed on light microscopy. However, the inventors found that many surviving spinal cord neurons from affected dogs accumulated cytoplasmic inclusions which were strongly stained by anti-SOD1 antibodies; whereas, only light and diffuse SOD1 staining was found in the spinal cords from age-matched control dogs. Light microscopic findings in spinal cords from affected dogs as well as clinical signs were comparable to the upper motor neuron-onset form of ALS. Furthermore, inclusions in the spinal cords from these dogs were similar to those found in ALS patients with *SOD1* mutations and in transgenic h*SOD1*$^{m}$ rodent models.

DM appears to be an incompletely penetrant autosomal recessive disease; whereas, most human *SOD1* mutations cause dominant forms of ALS. Nonetheless, the N90A SOD1 variant is associated with a recessively inherited form of ALS in some families but a dominantly inherited form of ALS in others (Andersen *et al.,* 1996). Among the families segregating for the dominant form of ALS, patients with two copies of the *SOD1* mutant generally have a much earlier age at disease onset than patients inheriting only a single copy (Hayward *et al.,* 1998; Marucci *et al.,* 2007). Furthermore, h*SOD1*$^{m}$ mice with higher transgene copy numbers exhibit earlier onset disease; and, the disease occurs much earlier in homozygous than heterozygous h*SOD1*$^{m}$ mice (Jonsson *et al.,* 2006). In these examples, the onset of clinical signs was inversely related to the copy number of the mutant *SOD1*. With canine DM the age at onset for SOD1:c.118G>A heterozygotes may exceed the normal canine life expectancy. In this case, the mode of inheritance would appear to be recessive even if the pathogenesis involves a gain of function.

The discovery that the E40K missense mutation is a major genetic risk factor underlying canine DM should enable dog breeders to use marker-based breeding to avoid producing future generations of dogs at risk for developing DM. Nonetheless, affected dogs born before the availability of a DM marker test will continue to develop clinical signs. Therefore, it will take at least a decade before marker-based breeding can substantially reduce the incidence of this late onset disease. In the mean time, many thousands of privately-owned affected dogs will continue to suffer from DM. It is hoped that future investigations of affected dogs will yield a better understanding of the disease processes underlying both canine DM and human ALS and will ultimately lead to therapeutic interventions of benefit to both human and canine patients. In this regard, a wide variety of potential therapeutic agents have been found to influence the age at onset and/or the rate of disease progression in h*SOD1*$^{m}$ mouse models; however, these agents have seldom performed well in human clinical trials (Benatar, 2007; Orrell *et al.,* 2007). Compared to the h*SOD1*$^{m}$ mouse model, dogs with DM are more similar to humans in size, in the structure of their nervous system, and in the duration of the disease. In addition, they are unlikely to possess the very high levels of SOD1 expression produced by many of the h*SOD1*$^{m}$ mouse models. Thus, the results from clinical trials conducted with DM dogs may better predict the success of therapeutic interventions for treating ALS.

All of the compositions and methods disclosed and/or claimed herein can be made and executed without undue experimentation in light of the present disclosure. While the compositions and methods disclosed have been described in terms of preferred embodiments, it will be apparent to those of skill in the art that variations may be applied to the compositions and methods, and in the steps or in the sequence of steps of the methods described herein. More specifically, it will be apparent that certain agents which are both chemically and physiologically related may be substituted for the agents described herein while the same or similar results would be achieved.

VI. References

The following references, to the extent that they provide exemplary procedural or other details supplementary to those set forth herein, are disclosed herein.


U.S. Patent 3,817,837
U.S. Patent 3,850,752
U.S. Patent 3,939,350
U.S. Patent 3,996,345
U.S. Patent 4,196,265
U.S. Patent 4,275,149
U.S. Patent 4,277,437
U.S. Patent 4,366,241
U.S. Patent 4,582,788
U.S. Patent 4,656,127
U.S. Patent 4,659,774
U.S. Patent 4,682,195
U.S. Patent 4,683,194
U.S. Patent 4,683,195
U.S. Patent 4,683,202
U.S. Patent 4,800,159
U.S. Patent 4,816,571
U.S. Patent 4,883,750
U.S. Patent 4,946,773
U.S. Patent 4,959,463
U.S. Patent 4,965,188
U.S. Patent 5,130,238
U.S. Patent 5,141,813
U.S. Patent 5,169,766
U.S. Patent 5,264,566
U.S. Patent 5,279,721
U.S. Patent 5,428,148
U.S. Patent 5,554,744
U.S. Patent 5,574,146
U.S. Patent 5,602,244
U.S. Patent 5,605,798
U.S. Patent 5,645,897
U.S. Patent 5,705,629

U.S. Patent 5,840,873
U.S. Patent 5,843,640
U.S. Patent 5,843,651
U.S. Patent 5,846,708
U.S. Patent 5,846,717
U.S. Patent 5,846,726
U.S. Patent 5,846,729
U.S. Patent 5,849,487
U.S. Patent 5,853,990
U.S. Patent 5,853,992
U.S. Patent 5,853,993
U.S. Patent 5,856,092
U.S. Patent 5,861,244
U.S. Patent 5,863,732
U.S. Patent 5,863,753
U.S. Patent 5,866,331
U.S. Patent 5,905,024
U.S. Patent 5,910,407
U.S. Patent 5,912,124
U.S. Patent 5,912,145
U.S. Patent 5,919,630
U.S. Patent 5,925,517
U.S. Patent 5,928,862
U.S. Patent 5,928,869
U.S. Patent 5,929,227
U.S. Patent 5,932,413
U.S. Patent 5,935,791
U.S. Patent 5,843,650
U.S. Patent 5,843,663
U.S. Patent 5,846,709
U.S. Patent 5,846,783
U.S. Patent 5,849,481
U.S. Patent 5,849,483
U.S. Patent 5,849,486
U.S. Patent 5,849,497
U.S. Patent 5,849,546
U.S. Patent 5,849,547
U.S. Patent 5,851,770
U.S. Patent 5,851,772
U.S. Patent 5,858,652
U.S. Patent 5,866,337
U.S. Patent 5,866,366
U.S. Patent 5,900,481
U.S. Patent 5,912,148
U.S. Patent 5,916,776
U.S. Patent 5,916,779
U.S. Patent 5,919,626
U.S. Patent 5,922,574
U.S. Patent 5,925,525
U.S. Patent 5,928,870
U.S. Patent 5,928,905
U.S. Patent 5,928,906
U.S. Patent 5,932,451
U.S. Patent 5,935,825
U.S. Patent 5,939,291
U.S. Patent 5,942,391
U.S. Patent 5,952,174
Abbondanzo et al., Breast Cancer Res. Treat., 16:182(151), 1990.

Allred et al., Arch. Surg., 125(1):107-113, 1990.
Andersen et al., Brain, 119:1153-1172, 1996.
Ausubel et al., In: Current Protocols in Molecular Biology, John, Wiley & Sons, Inc, New York, 1989.
Barany, et al., Proc. Natl. Acad. Sci. USA, 88:189-193, 1991
Barrett et al., Bioinformatics, 21:263-265, 2005.
Bellus, J. Macromol. Sci. Pure Appl. Chem., A31(1): 1355-1376, 1994.
Benatar, Neurobiol. Disease, 26:1-13, 2007.
Benatar, Neuromusc. Disorders, 17:671-672, 2007.
Boissinot et al., EMBO J., 16:2171-2178, 1997.
Brown et al., Immunol Ser, 53:69-82, 1990.
Capaldi et al., Biochem. Biophys. Res. Comm., 74(2):425-433, 1977.
de Arruda et al., Expert Rev. Mol. Diagn., 2(5):487-496, 2002.
De Jager et al., Semin. Nucl. Med., 23(2):165-179, 1993.
Deng et al., Science, 261:1047-1051, 1993.
Doolittle et al., Methods Mol. Biol., 109:215-237, 1999.
EP 201,184
EP 237,362
EP 258,017
EP 266,032
EP 329,822
EP 50,424
EP 84,796
Fogh et al., Neurogenet., 8:235-236, 2007.
French Patent 2,650,840
Froehler et al., Nucleic Acids Res., 14(13):5399-5407, 1986.
Frohman, In: PCR Protocols: A Guide To Methods And Applications, Academic Press, N.Y., 1990.
Great Britain Appln. 2 202 328
Green et al., J. Hered., 93:119-124, 2002.
Gulbis and Galand, Hum. Pathol., 24(12):1271-1285, 1993.
Halushka et al., Nat. Genet., 22(3):239-247,1999.
Harlow and Lane, In: Antibodies: A Laboratory Manual, Cold Spring Harbor Laboratory, 1988.
Hayward et al., J. Med. Genet., 35:174, 1998.
Heiman-Patterson et al., J. Neurolog. Sci., 236:1-7, 2005.
Humphries et al., In: Molecular Diagnosis of Genetic Diseases, Elles (Ed.), 321-340, 1996.
Inazuka et al., Genome Res, 7(11):1094-1103, 1997.
Innis et al, Proc. Natl. Acad. Sci. USA, 85(24):9436-9440, 1988.
Johnson et al., Nat. Genet., 29(2):233-237, 2001.
Jones, Nature, 199:280-282, 1963.
Jonsson et al., J. Neuropathol. Exp. Neurol., 65:1126-1136, 2006.
Karlsson et al., Nat. Genet., 39:1321-1328, 2007.
Ke and Cardon Bioinformatics, 19(2):287-288, 2003.
Komher, et al., Nucl. Acids. Res. 17:7779-7784, 1989.
Kuppuswamy et al., Proc. Natl. Acad. Sci. USA, 88:1143-1147,1991.
Kwoh et al., Proc. Natl. Acad. Sci. USA, 86:1173, 1989.
Kwok and Chen, Curr Issues Mol. Biol., Apr;5(2):43-60, 2003.
Kwok et al., Genomics, 23(1):138-144, 1994.
Kwok et al., Genomics, 31(1):123-6, 1996.
Kwok, Annu. Rev. Genomics Hum. Genet., 2:235-258, 2001.
Landegren et al., Science 241:1077-1080, 1988.
Lindberg et al., Proc. Natl. Acad. Sci. USA, 102:9754-9759, 2005.
Lu et al., Biopolymers, 73:606-613, 2004.
Marucci et al., Neuromuscul. Disord., 17:673-676, 2007.
Maxam et al., Proc. Natl. Acad. Sci. USA, 74:560, 1977.
Meyers et al., Science, 230:1242, 1985.
Modrich, Ann. Rev. Genet., 25:229-253, 1991.
Mullis et al., Cold Spring Harbor Symp. Quant. Biol. 51:263-273, 1986.
Nakamura et al., In: Handbook of Experimental Immunology (4th Ed.), Weir et al., (eds). 1:27, Blackwell Scientific Publ., Oxford, 1987.

Nickerson et al., Proc. Natl. Acad. Sci. USA, 87:8923-8927,1990.
Nyren *et al.,* 1993).
Nyren et al., Anal. Biochem. 208:171-175, 1993.
Ohara et al., Proc. Natl. Acad. Sci. USA, 86:5673-5677, 1989.
Orrell et al., N. Eng. J. Med., 357:822-823, 2007.
PCT Appln. PCT/US87/00880
PCT Appln. PCT/US89/01025
PCT Appln. WO 88/10315
PCT Appln. WO 89/06700
PCT Appln. WO 89/06700
PCT Appln. WO 90/01069
PCT Appln. WO 91/02087
PCT Appln. WO 92/15712
PCT Appln. WO 93/22456
PCT Appln. WO 95/11995
Prezant et al., Hum. Mutat., 1:159-164, 1992.
Purcell et al., Am. J. Hum. Genet., 81:559-575, 2007.
Ruano et al., Nucl. Acids Res., 19:6877-6882, 1991.
Ruano et al., Nucl. Acids Res., 17:8392, 1989.
Sambrook et al., In: Molecular cloning, Cold Spring Harbor Laboratory Press, Cold Spring Harbor, NY, 2001.
Sandelin, et al. J. Biol. Chem., 282:21230-21236, 2007.
Sanger et al., J. Molec. Biol., 94:441, 1975.
Shaw et al., Trends Biochem. Sci., 32, 78-85, 2007.
Sheffield et al., Proc. Natl. Acad. Sci. USA, 86:232-236, 1989.
Sokolov, Nucl. Acids Res. 18:3671, 1990.
Stevens et al., Biotechniques, 34:198-203, 2003.
Syvanen et al., Genomics 8:684-692, 1990.
Taillon-Miller et al., Genome Res, 8(7):748-754, 1998.
Tainer et al., J. Mol. Biol., 160:181-217, 1982.
Turki et al., J. Clin. Invest., 95:1635-1641, 1995.
Ugozzoll et al., GATA 9:107-112, 1992.
Valentine et al., Annu. Rev. Biochem., 74:563-593, 2005.
Walker et al., Am. J. Vet. Res. 40:1433-1439, 1979.
Walker et al., Proc. Natl. Acad. Sci. USA, 89:392-396, 1992.
Wartell et al., Nucl. Acids Res., 18:2699-2706, 1990.
Winter et al., Proc. Natl. Acad. Sci. USA, 82:7575, 1985.

## Claims

1. A method of identifying a dog as having or at risk of developing degenerative myelopathy, or producing offspring having or at risk of developing degenerative myelopathy, comprising:

   (a) providing a nucleic acid-containing sample from said dog;
   (b) assessing the structure of an SOD1 gene or transcript corresponding to amino acid residue 40 of the corresponding SOD1 polypeptide, in said nucleic acid-containing sample from said dog; and
   (c) identifying said dog as (i) having or at risk of developing degenerative myelopathy when a polymorphism in the SOD1 coding region corresponding to a homozygous E → K substitution at amino acid residue 40 of the corresponding SOD1 polypeptide, as compared to a reference wild-type SOD1 gene or transcript is observed, or (ii) producing offspring having or at risk of developing degenerative myelopathy when a polymorphism in the SOD1 coding region corresponding to a heterozygous E → K substitution at amino acid residue 40 of the corresponding SOD1 polypeptide, as compared to a reference wild-type SOD1 gene or transcript is observed.

2. The method of claim 1, wherein said dog is a boxer, a Welsh corgi, a Chesapeake Bay retriever, a Rhodesian ridgeback, a German shepherd, a Kerry blue terrier, a Irish setter, a old English sheepdog, a collie, a standard poodle, a wire Fox terrier or another breed, or a cross-breed thereof.

3. The method of claim 1, wherein step (b) comprises pyrosequencing, chain-terminating sequencing, restriction digestion, allele-specific polymerase reaction, single-stranded conformational polymorphism analysis, genetic bit analysis, temperature gradient gel electrophoresis, ligase chain reaction, melting curve profiles, TaqMan® allelic discrimination assay, or microarray hybridization.

4. The method of claim 1, wherein the nucleic acid-containing sample comprises blood, buccal tissue, skin, semen, or hair follicles from said dog.

5. The method of claim 1, wherein said reference wild-type SOD1 gene comprises SEQ ID NO:1.

## Patentansprüche

1. Verfahren zum Identifizieren eines Hundes als degenerative Myelopathie habend oder ein Risiko habend, degenerative Myelopathie zu entwickeln, oder einen Nachkommen erzeugend, der degenerative Myelopathie hat oder ein Risiko hat, degenerative Myelopathie zu entwickeln, aufweisend:

   (a) Bereitstellen einer Nukleinsäure enthaltenden Probe von dem Hund;
   (b) Beurteilen der Struktur eines SOD1-Genes oder -Transkriptes, das mit dem Aminosäurerest 40 des korrespondierenden SOD1-Polypeptides korrespondiert, in der Nukleinsäure enthaltenden Probe von dem Hund; und
   (c) Identifizieren des Hundes als (i) degenerative Myelopathie habend oder ein Risiko habend, degenerative Myelopathie zu entwickeln, wenn ein Polymorphismus in der SOD1-Kodierungsregion, der mit einer homozygoten E->K-Substitution bei dem Aminosäurerest 40 des korrespondierenden SOD1-Polypeptides korrespondiert, im Vergleich zu einem Wildtyp-Referenz-SOD1-Gen oder -Transkript beobachtet wird oder (ii) einen Nachkommen erzeugend, der degenerative Myelopathie hat oder ein Risiko hat, degenerative Myelopathie zu entwickeln, wenn ein Polymorphismus in der SOD1-Kodierungsregion, der mit einer heterozygoten E->K-Substitution bei dem Aminosäurerest 40 des korrespondierenden SOD1-Polypeptides korrespondiert, im Vergleich zu einem Wildtyp-Referenz-SOD1-Gen oder -Transkript beobachtet wird.

**2.** Verfahren nach Anspruch 1, wobei der Hund ein Boxer, ein Welsh Corgi, ein Chesapeake Bay Retriever, ein Rhodesian Ridgeback, ein deutscher Schäferhund, ein Kerry Blue Terrier, ein Irish Setter, ein alter englischer Schäferhund, ein Collie, ein Standard-Pudel, ein Rauhaar-Foxterrier oder eine andere Rasse oder eine Kreuzung davon ist.

**3.** Verfahren nach Anspruch 1, wobei Schritt (b) Pyrosequenzierung, Kettenabbruchsequenzierung, Restriktionsabbau, Allel-spezifische Polymerasereaktion, Einzelstrangkonformationspolymorphismus-Analyse, genetische Bit-Analyse, Temperaturgradienten-Gelelektrophorese, Ligase-Kettenreaktion, Schmelzkurvenprofile, TaqMan®-Allelunterscheidungsassay oder Mikroarrayhybridisierung aufweist.

**4.** Verfahren nach Anspruch 1, wobei die Nukleinsäure enthaltende Probe Blut, bukkales Gewebe, Haut, Sperma oder Haarfollikel von dem Hund aufweist.

**5.** Verfahren nach Anspruch 1, wobei das Wildtyp-Referenz-SOD1-Gen SEQ ID-Nr. 1 aufweist.

## Revendications

**1.** Procédé d'identification d'un chien comme souffrant de ou à risque de développer une myélopathie dégénérative, ou produisant une descendance souffrant de ou à risque de développer une myélopathie dégénérative, comprenant :

(a) la fourniture d'un échantillon contenant de l'acide nucléique provenant dudit chien ;
(b) l'estimation de la structure d'un gène SOD1 ou d'un transcrit correspondant au résidu d'acide aminé 40 du polypeptide SOD1 correspondant, dans ledit échantillon contenant de l'acide nucléique provenant dudit chien ; et
(c) l'identification dudit chien comme (i) souffrant de ou à risque de développer une myélopathie dégénérative lorsqu'un polymorphisme dans la région codante de SOD1 correspondant à une substitution homozygote E $\rightarrow$ K au niveau du résidu d'acide aminé 40 du polypeptide SOD1 correspondant, comparativement à un gène SOD1 de type sauvage de référence ou à un transcrit, est observé, ou (ii) produisant une descendance souffrant de ou à risque de développer une myélopathie dégénérative lorsqu'un polymorphisme dans la région codante de SOD1 correspondant à une substitution hétérozygote E $\rightarrow$ K au niveau du résidu d'acide aminé 40 du polypeptide SOD1 correspondant, comparativement à un gène SOD1 de type sauvage de référence ou à un transcrit, est observé.

**2.** Procédé selon la revendication 1, dans lequel ledit chien est un boxer, un Welsh Corgi, un Retriever de la Baie de Chesapeake, un Rhodesian Ridgeback, un berger allemand, un Kerry Blue Terrier, un setter irlandais, un berger ancestral anglais, un Collie, un caniche de taille standard, un fox-terrier à poil dur ou une autre race, ou un croisement de ceux-ci.

**3.** Procédé selon la revendication 1, dans lequel l'étape (b) comprend un pyroséquençage, un séquençage par terminaison de chaîne, une digestion par des enzymes de restriction, une réaction de la polymérase spécifique de l'allèle, l'analyse d'un polymorphisme conformationnel simple brin, une analyse de bits génétiques, une électrophorèse sur gel à gradient de température, une réaction en chaîne de la ligase, des profils de courbes de fusion, le test de discrimination allélique Taqman® ou une hybridation sur micropuce.

**4.** Procédé selon la revendication 1, dans lequel l'échantillon contenant de l'acide nucléique comprend le sang, le tissu buccal, la peau, le sperme ou des follicules pileux provenant dudit chien.

**5.** Procédé selon la revendication 1, dans lequel ledit gène SOD1 de type sauvage de référence comprend SÉQ ID NO : 1.

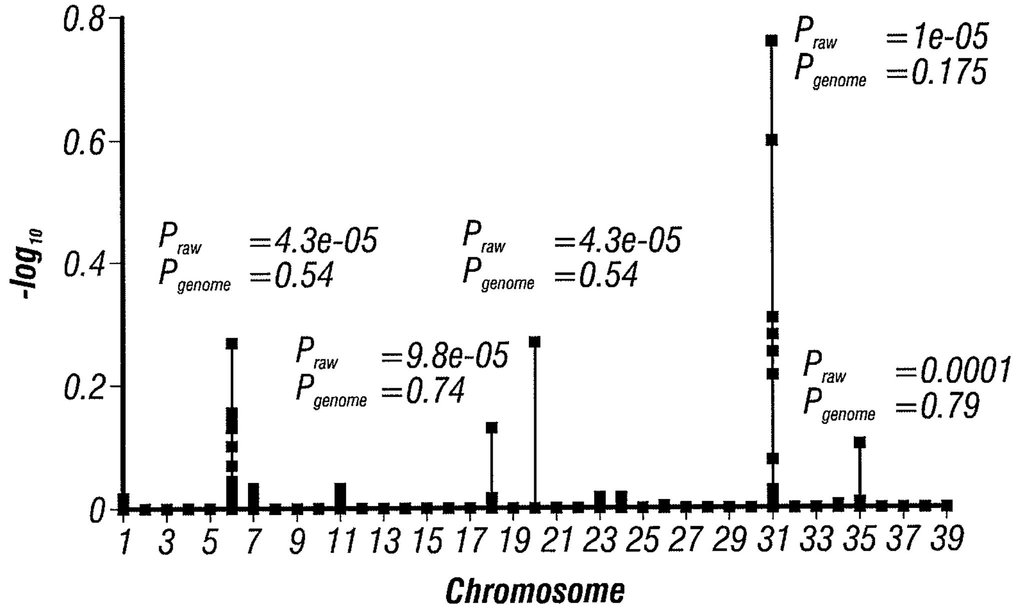
0.8
0.6
0.4
0.2
0
-log10
P raw =1e-05
P genome =0.175
P raw =4.3e-05
P genome =0.54
P raw =4.3e-05
P genome =0.54
P raw =9.8e-05
P genome =0.74
P raw =0.0001
P genome =0.79
1 3 5 7 9 11 13 15 17 19 21 23 25 27 29 31 33 35 37 39
Chromosome

FIG. 1A

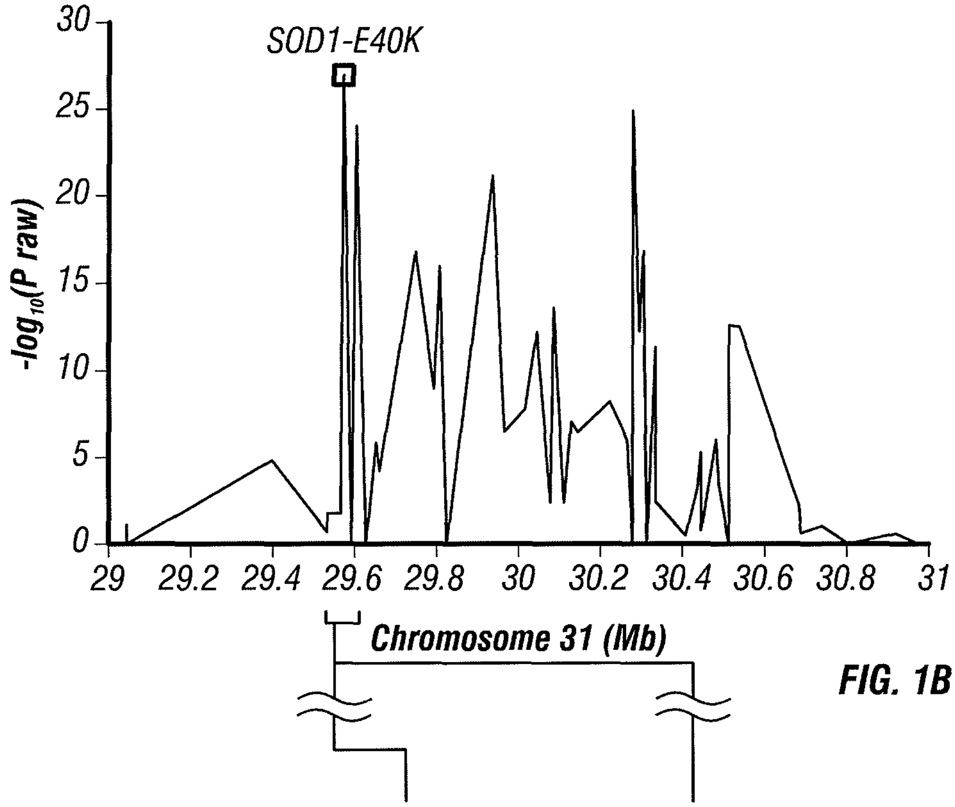


**FIG. 1B**

| | | SOD-1 mut. (%) | | SOD-1 haplotype (%) | | |
|---|---|---|---|---|---|---|
| CASES | N | A | G | GTTAC | ACCGT | Other |
| Pembroke Welsh Corgi | 35 | 100 | 0 | 100 | 0 | 0 |
| Boxer | 8 | 100 | 0 | 100 | 0 | 0 |
| Rhodesian Ridgeback | 6 | 100 | 0 | 100 | 0 | 0 |
| Chesapeake Bay Retriever | 9 | 100 | 0 | 100 | 0 | 0 |
| German Shepherd | 4 | 100 | 0 | 100 | 0 | 0 |
| CONTROLS | | | | | | |
| Pembroke Welsh Corgi | 17 | 74 | 26 | 88 | 12 | 0 |
| Boxer | 15 | 67 | 33 | 100 | 0 | 0 |
| Rhodesian Ridgeback | 10 | 15 | 85 | 95 | 0 | 5 |
| Chesapeake Bay Retriever | 48 | 39 | 61 | 71 | 23 | 6 |
| German Shepherd | 53 | 25 | 75 | 98 | 2 | 0 |

**FIG. 1C**

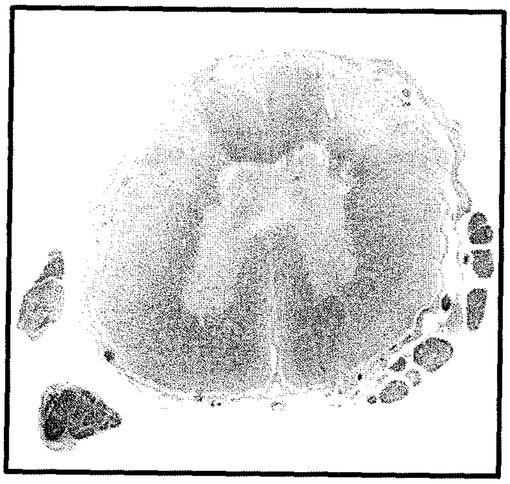

FIG. 2A

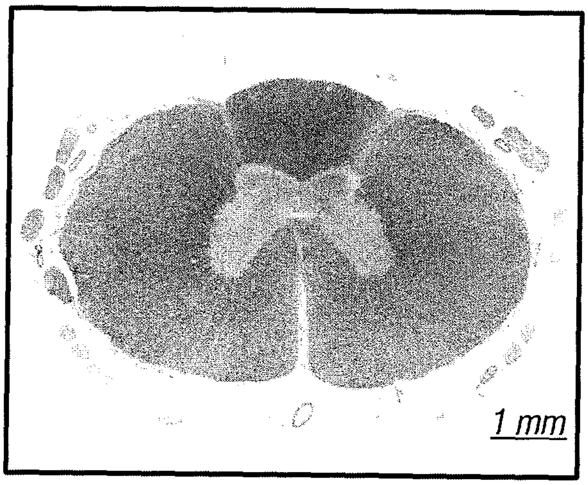


FIG. 2B

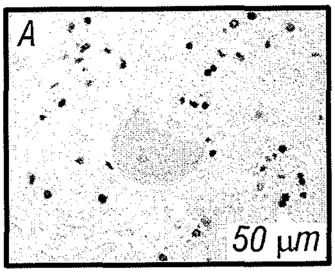


FIG. 3A

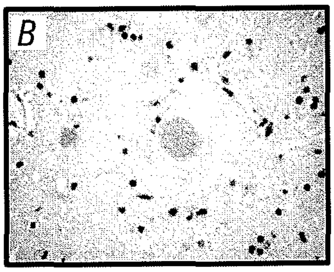


FIG. 3B

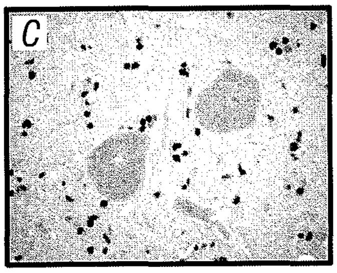


FIG. 3C

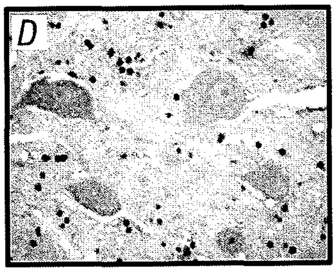


FIG. 3D

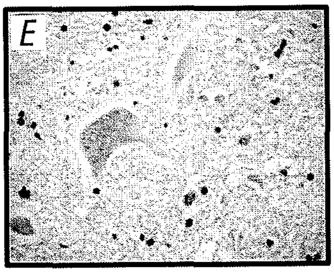


FIG. 3E

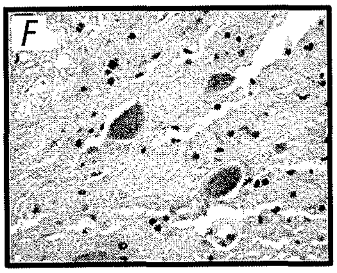


FIG. 3F

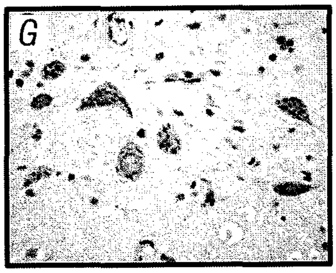


FIG. 3G

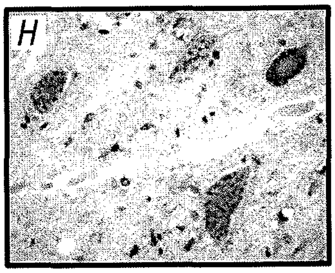


FIG. 3H

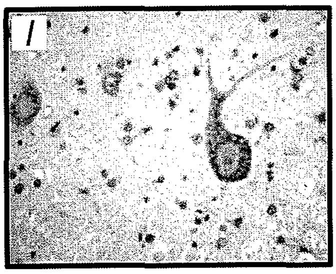


FIG. 3I

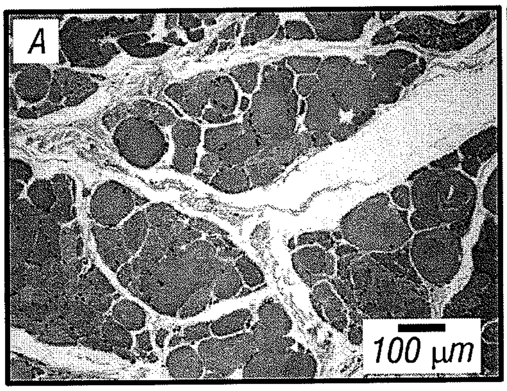


FIG. 4A

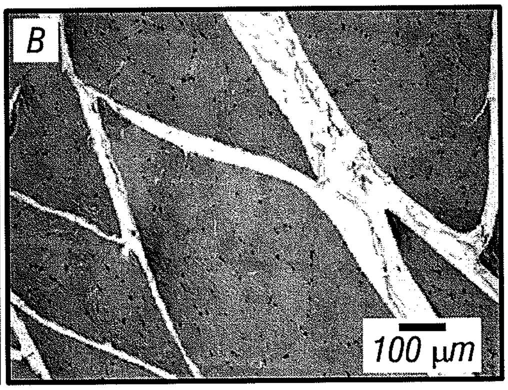


FIG. 4B

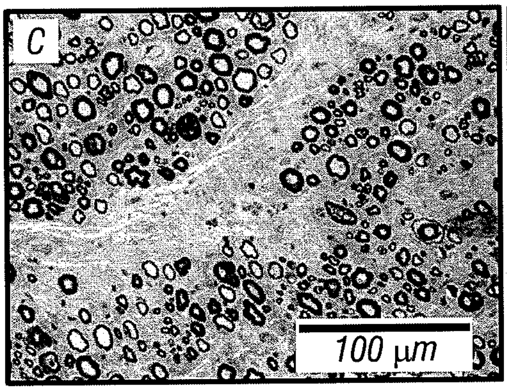


FIG. 4C

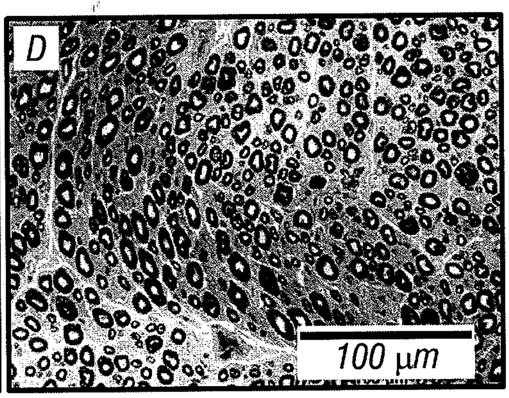


FIG. 4D

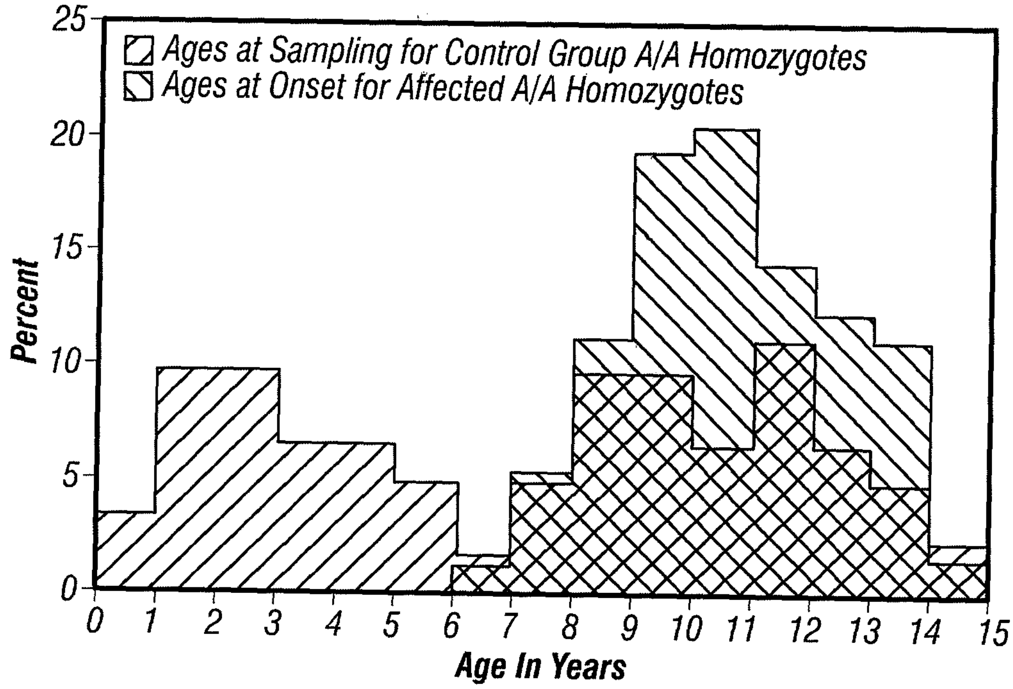
25
20
15
10
5
0
Percent
Ages at Sampling for Control Group A/A Homozygotes
Ages at Onset for Affected A/A Homozygotes
0 1 2 3 4 5 6 7 8 9 10 11 12 13 14 15
Age In Years

FIG. 5

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 266032 A
- US 5705629 A
- US 4659774 A
- US 4816571 A
- US 5141813 A
- US 5264566 A
- US 4959463 A
- US 5428148 A
- US 5554744 A
- US 5574146 A
- US 5602244 A
- US 4683202 A
- US 4682195 A
- US 5645897 A
- US 4965188 A
- WO 9001069 A
- US 5130238 A
- EP 329822 A
- US 5169766 A
- WO 8906700 A
- WO 9511995 A
- US 5605798 A
- WO 9322456 A
- US 5843663 A
- US 5900481 A
- US 5919626 A
- US 5849481 A
- US 5849486 A
- US 5851772 A
- US 4683195 A
- US 4800159 A
- EP 320308 A
- US 4883750 A
- US 5912148 A
- US 5843650 A
- US 5846709 A
- US 5846783 A
- US 5849546 A
- US 5849497 A
- US 5849547 A
- US 5858652 A
- US 5866366 A
- US 5916776 A
- US 5922574 A
- US 5928905 A
- US 5928906 A
- US 5932451 A
- US 5935825 A
- US 5939291 A
- US 5942391 A
- GB 2202328 A
- US 8901025 W
- US 8700880 W
- US 5916779 A
- WO 8810315 A
- US 5279721 A
- US 5840873 A
- US 5843640 A
- US 5843651 A
- US 5846708 A
- US 5846717 A
- US 5846726 A
- US 5846729 A
- US 5849487 A
- US 5853990 A
- US 5853992 A
- US 5853993 A
- US 5856092 A
- US 5861244 A
- US 5863732 A
- US 5863753 A
- US 5866331 A
- US 5905024 A
- US 5910407 A
- US 5912124 A
- US 5912145 A
- US 5919630 A
- US 5925517 A
- US 5928862 A
- US 5928869 A
- US 5929227 A
- US 5932413 A
- US 5935791 A
- US 4946773 A
- US 5849483 A
- US 5851770 A
- US 5866337 A
- US 5925525 A
- US 5928870 A
- EP 50424 A
- EP 84796 A
- EP 258017 A
- EP 237362 A
- EP 201184 A
- US 4582788 A
- US 4683194 A
- US 4656127 A
- FR 2650840

- WO 9102087 A
- WO 9215712 A
- US 5952174 A
- US 4196265 A
- US 3817837 A
- US 3850752 A
- US 3939350 A
- US 3996345 A
- US 4275149 A
- US 4277437 A
- US 4366241 A
- US 61025949 B

**Non-patent literature cited in the description**


- **GREEN et al.** *The American Genetic Association,* 2002, vol. 93, 119-124
- **ABBONDANZO et al.** *Breast Cancer Res. Treat.,* 1990, vol. 182 (151
- **ALLRED et al.** *Arch. Surg.,* 1990, vol. 125 (1), 107-113
- **ANDERSEN et al.** *Brain,* 1996, vol. 119, 1153-1172
- **AUSUBEL et al.** Current Protocols in Molecular Biology. John, Wiley & Sons, Inc, 1989
- **BARANY et al.** *Proc. Natl. Acad. Sci. USA,* 1991, vol. 88, 189-193
- **BARRETT et al.** *Bioinformatics,* 2005, vol. 21, 263-265
- **BELLUS.** *J. Macromol. Sci. Pure Appl. Chem.,* 1994, vol. A31 (1), 1355-1376
- **BENATAR.** *Neurobiol. Disease,* 2007, vol. 26, 1-13
- **BENATAR.** *Neuromusc. Disorders,* 2007, vol. 17, 671-672
- **BOISSINOT et al.** *EMBO J.,* 1997, vol. 16, 2171-2178
- **BROWN et al.** *Immunol Ser,* 1990, vol. 53, 69-82
- **CAPALDI et al.** *Biochem. Biophys. Res. Comm.,* 1977, vol. 74 (2), 425-433
- **DE ARRUDA et al.** *Expert Rev. Mol. Diagn.,* 2002, vol. 2 (5), 487-496
- **DE JAGER et al.** *Semin. Nucl. Med.,* 1993, vol. 23 (2), 165-179
- **DENG et al.** *Science,* 1993, vol. 261, 1047-1051
- **DOOLITTLE et al.** *Methods Mol. Biol.,* 1999, vol. 109, 215-237
- **FOGH et al.** *Neurogenet.,* 2007, vol. 8, 235-236
- **FROEHLER et al.** *Nucleic Acids Res.,* 1986, vol. 14 (13), 5399-5407
- **FROHMAN.** PCR Protocols: A Guide To Methods And Applications. Academic Press, 1990
- **GREEN et al.** *J. Hered.,* 2002, vol. 93, 119-124
- **GULBIS ; GALAND.** *Hum. Pathol.,* 1993, vol. 24 (12), 1271-1285
- **HALUSHKA et al.** *Nat. Genet.,* 1999, vol. 22 (3), 239-247
- **HARLOW ; LANE.** Antibodies: A Laboratory Manual. Cold Spring Harbor Laboratory, 1988
- **HAYWARD et al.** *J. Med. Genet.,* 1998, vol. 35, 174
- **HEIMAN-PATTERSON et al.** *J. Neurolog. Sci.,* 2005, vol. 236, 1-7
- **HUMPHRIES et al.** Molecular Diagnosis of Genetic Diseases. 1996, 321-340
- **INAZUKA et al.** *Genome Res,* 1997, vol. 7 (11), 1094-1103
- **INNIS et al.** *Proc. Natl. Acad. Sci. USA,* 1988, vol. 85 (24), 9436-9440
- **JOHNSON et al.** *Nat. Genet.,* 2001, vol. 29 (2), 233-237
- **JONES.** *Nature,* 1963, vol. 199, 280-282
- **JONSSON et al.** *J. Neuropathol. Exp. Neurol.,* 2006, vol. 65, 1126-1136
- **KARLSSON et al.** *Nat. Genet.,* 2007, vol. 39, 1321-1328
- **KE ; CARDON.** *Bioinformatics,* 2003, vol. 19 (2), 287-288
- **KOMHER et al.** *Nucl. Acids. Res.,* 1989, vol. 17, 7779-7784
- **KUPPUSWAMY et al.** *Proc. Natl. Acad. Sci. USA,* 1991, vol. 88, 1143-1147
- **KWOH et al.** *Proc. Natl. Acad. Sci. USA,* 1989, vol. 86, 1173
- **KWOK ; CHEN.** *Curr Issues Mol. Biol.,* 2003, vol. 5 (2), 43-60
- **KWOK et al.** *Genomics,* 1994, vol. 23 (1), 138-144
- **KWOK et al.** *Genomics,* 1996, vol. 31 (1), 123-6
- **KWOK.** *Annu. Rev. Genomics Hum. Genet.,* 2001, vol. 2, 235-258
- **LANDEGREN et al.** *Science,* 1988, vol. 241, 1077-1080
- **LINDBERG et al.** *Proc. Natl. Acad. Sci. USA,* 2005, vol. 102, 9754-9759
- **LU et al.** *Biopolymers,* 2004, vol. 73, 606-613
- **MARUCCI et al.** *Neuromuscul. Disord.,* 2007, vol. 17, 673-676
- **MAXAM et al.** *Proc. Natl. Acad. Sci. USA,* 1977, vol. 74, 560
- **MEYERS et al.** *Science,* 1985, vol. 230, 1242
- **MODRICH.** *Ann. Rev. Genet.,* 1991, vol. 25, 229-253
- **MULLIS et al.** *Cold Spring Harbor Symp. Quant. Biol.,* 1986, vol. 51, 263-273

- **NAKAMURA et al.** Handbook of Experimental Immunology. Blackwell Scientific Publ, 1987, vol. 1, 27
- **NICKERSON et al.** *Proc. Natl. Acad. Sci. USA,* 1990, vol. 87, 8923-8927
- **NYREN et al.** *Anal. Biochem.,* 1993, vol. 208, 171-175
- **OHARA et al.** *Proc. Natl. Acad. Sci. USA,* 1989, vol. 86, 5673-5677
- **ORRELL et al.** *N. Eng. J. Med.,* 2007, vol. 357, 822-823
- **PREZANT et al.** *Hum. Mutat.,* 1992, vol. 1, 159-164
- **PURCELL et al.** *Am. J. Hum. Genet.,* 2007, vol. 81, 559-575
- **RUANO et al.** *Nucl. Acids Res.,* 1991, vol. 19, 6877-6882
- **RUANO et al.** *Nucl. Acids Res.,* 1989, vol. 17, 8392
- **SAMBROOK et al.** Molecular cloning. Cold Spring Harbor Laboratory Press, 2001
- **SANDELIN et al.** *J. Biol. Chem.,* 2007, vol. 282, 21230-21236
- **SANGER et al.** *J. Molec. Biol.,* 1975, vol. 94, 441
- **SHAW et al.** *Trends Biochem. Sci.,* 2007, vol. 32, 78-85
- **SHEFFIELD et al.** *Proc. Natl. Acad. Sci. USA,* 1989, vol. 86, 232-236
- **SOKOLOV.** *Nucl. Acids Res.,* 1990, vol. 18, 3671
- **STEVENS et al.** *Biotechniques,* 2003, vol. 34, 198-203
- **SYVANEN et al.** *Genomics,* 1990, vol. 8, 684-692
- **TAILLON-MILLER et al.** *Genome Res,* 1998, vol. 8 (7), 748-754
- **TAINER et al.** *J. Mol. Biol.,* 1982, vol. 160, 181-217
- **TURKI et al.** *J. Clin. Invest.,* 1995, vol. 95, 1635-1641
- **UGOZZOLL et al.** *GATA,* 1992, vol. 9, 107-112
- **VALENTINE et al.** *Annu. Rev. Biochem.,* 2005, vol. 74, 563-593
- **WALKER et al.** *Am. J. Vet. Res.,* 1979, vol. 40, 1433-1439
- **WALKER et al.** *Proc. Natl. Acad. Sci. USA,* 1992, vol. 89, 392-396
- **WARTELL et al.** *Nucl. Acids Res.,* 1990, vol. 18, 2699-2706
- **WINTER et al.** *Proc. Natl. Acad. Sci. USA,* 1985, vol. 82, 7575